# EUROPEAN PATENT APPLICATION

(11) **EP 2 623 991 A1**
(43) Date of publication of application: **07.08.2013**
(21) Application number: 11829279.6
(22) Date of filing: 29.09.2011
(51) Int. Cl.: G01N 35/08, B01J 19/00, B23K 26/00, B81B 1/00, B81C 1/00, G01N 37/00, C12M 1/00, G01N 27/28

(54) **BASE BODY AND METHOD FOR MANUFACTURING BASE BODY**

(30) Priority: 30.09.2010 JP 2010222225
(71) Applicant: Fujikura Co., Ltd., Tokyo 135-8512 (JP); The University of Tokyo, Bunkyo-ku, Tokyo 113-8654 (JP)
(72) Inventor: NUKAGA Osamu, Sakura-shi Chiba 285-8550 (JP); YAMAMOTO Satoshi, Sakura-shi Chiba 285-8550 (JP); TABATA Kazuhito, Tokyo 113-8654 (JP); SUGIYAMA Masakazu, Tokyo 113-8654 (JP)
(74) Representative: Cabinet Plasseraud
(86) International application number: PCT/JP2011/072392
(87) International publication number: WO 2012/043726

(57) **Abstract**

A base body includes: a base member (4); a channel (3) provided in the base member (4), the channel having an inner wall surface and flowing a fluid; a fine vacuum hole (1) provided in the inner wall, the fine vacuum hole causing the channel (3) to communicate with the outside of the base member (4) and having an opening; and a slow flow portion (P) disposed at a position close to the opening of the fine vacuum hole (1) in the inner wall surface, the slow flow portion slows a flow of the fluid, wherein at least a portion that configures the fine vacuum hole in the base member is formed of a single member.

## Description

### TECHNICAL FIELD

The present invention relates to a base body including a fine vacuum hole that traps a fine particle, and a method for manufacturing a base body. Priority is claimed on Japanese Patent Application No. 2010-222225, filed September 30, 2010, the content of which is incorporated herein by reference.

### BACKGROUND ART

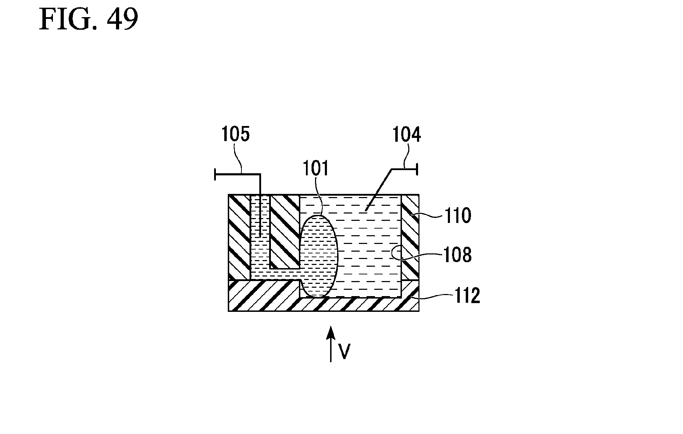

As a method for physically trapping cells collected from a living organism or cultured cells and performing an electrophysiological measurement of the cells, a patch-clamp method is known. As a method in the related art for physically trapping cells, a method (FIG. 49) for suctioning cells 101 through a hole provided at the lateral side of a resin well 108 is known (Non-Patent Document 1). Non-Patent Document 1 discloses a device capable of directly observing the behavior of cells trapped in the hole from the rear surface of the device using a high-power microscope, or capable of electrophysiologically measuring the cell membranes of cells and the like trapped in the hole. These serve as effective means as means for elucidating new biological properties.

Generally, in a patch-clamp method, it is required to achieve a strong seal (high-resistance seal) of 1 gigaohm or more by causing an adsorption portion (opening) of the hole and cell membranes to adhere closely to each other. In order to form such a high-resistance seal, it is necessary to stabilize the seal between the cell membranes and the adsorption portion, and an absorption portion with a very small diameter and a stepless or jointless hole shape are required.

A cell trapping device disclosed in Non-Patent Document 1 includes a base body in which two members formed of a resin (PDMS) are bonded to each other, and the bonding interface thereof is provided with a fine hole. The base body is provided with a space that holds a fluid. This space is formed by punching the base body. At the time of the punching, the fine hole formed within the base body beforehand is opened to the space.

Since the cell trapping device is manufactured by a method for punching a resin, it is difficult to create the shape of the adsorption portion (opening) of the fine hole for trapping cells in a desired shape. In addition, when a plurality of adsorption portions (openings) are formed in the base body, it is difficult to uniformly form the shapes of the plurality of adsorption portions (openings). In addition, since the base body is a resin, it is difficult to form a finer hole than a minor diameter of approximately 2 microns. Furthermore, since the fine hole is formed by bonding two members together, a joint or a step occurring by linking two members together is present in the adsorption portion. When such a joint or step is present, it may be difficult to stably trap cells. That is, since the sizes or shapes of the fine hole and the adsorption portion which are formed in the cell trapping device have a low accuracy, there is a problem in that it is difficult to trap cells as desired.

In addition, when the electrophysiological measurement is performed, there is a problem in that since the seal between the cell membrane and the adsorption portion becomes unstable, it is difficult to perform a high-accuracy electrophysiological measurement.
Furthermore, in order to trap microorganisms (having a short side on the order of nanometers) that are smaller than cells, it is necessary to highly accurately form the diameter of the short side of the adsorption portion (opening portion) of the micro holes at least on the order of nanometers. However, in Non-Patent Document 1, since a plurality of resin substrates 110 and 112 are configured to be bonded to each other, it is difficult to process the adsorption portion (opening) on the order of nanometers.

In order to stably perform the electrophysiological measurement and increase the degree of accuracy, it may be effective to reduce the area of the hole that traps cells. For this reason, it is desirable to develop a device including a hole with a diameter smaller than the diameter (approximately 2 µm to 4 µm) of the hole in the related art and a method for manufacturing the device.
In addition, it is desirable to develop a device by which fine particles including microorganisms or cells, which are contained in a fluid are trapped, and then can be observed, manipulated, measured and the like.

### PRIOR ART DOCUMENTS

### NON-PATENT DOCUMENTS

[Non-Patent Document 1] Adrian Y. Lau, et al. Lab Chip, 2006, 6, 1510-1515

### DISCLOSURE OF INVENTION

### PROBLEMS TO BE SOLVED BY INVENTION

The present invention in contrived in view of such circumstances, and an object thereof is to provide a base body in which a hole that traps a fine particle contained in a fluid is formed by a single base member and the trapped fine particle is easily observed, and a method for manufacturing the base body.

### MEANS FOR SOLVING THE PROBLEMS

A base body according to a first aspect of the present invention includes: a base member; a channel provided in the base member, the channel having an inner wall surface and circulating a fluid; a fine vacuum hole provided in the inner wall, the fine vacuum hole causing the channel to communicate with the outside of the base member and having an opening; and a slow flow portion, disposed at a position close to the opening of the fine vacuum hole in the inner wall surface, which slows the flow of the fluid, wherein at least a portion that configures the fine vacuum hole in the base member is formed of a single member.
In the base body according to the first aspect of the present invention, it is preferable that the inner wall surface of the channel include a first inner wall surface in which the slow flow portion is disposed and a second inner wall surface in which the opening of the fine vacuum hole is disposed, and the first inner wall surface and the second inner wall surface be different from each other.
In the base body according to the first aspect of the present invention, it is preferable that the inner wall surface of the channel include a first inner wall surface in which the slow flow portion is disposed and a second inner wall surface in which the opening of the fine vacuum hole is disposed, and the first inner wall surface be the same as the second inner wall surface.
In the base body according to the first aspect of the present invention, it is preferable that the slow flow portion be a recess portion formed in the inner wall surface.
In the base body according to the first aspect of the present invention, it is preferable to further include a protruding portion provided at a position close to the recess portion, the protruding portion protruding from an inner wall surface different from the inner wall surface in which the fine vacuum hole is formed.
In the base body according to the first aspect of the present invention, it is preferable that the recess portion include a first comer provided at a position close to the opening and a second corner provided at a position away from the opening, and in a longitudinal cross-section of the fine vacuum hole along an extending direction of the channel, when the distance between the first corner in a direction perpendicular to the extending direction of the channel and the opening is denoted by L, and the distance between the first corner in the extending direction of the channel and the opening is denoted by W, the ratio of L/W be in a range of 0.05 to 100.
A method for manufacturing a base body according to a second aspect of the present invention includes: a process A1 of preparing a base member including a single member; forming a first modified region by irradiating a region in which a fine vacuum hole is formed, in the single member, with a laser having a pulse duration of a picosecond order or less, a process A2 of forming a second modified region by irradiating a region in which a channel is formed, in the single member, with a laser having a pulse duration of a picosecond order or less, forming a third modified region by irradiating a region in which a slow flow portion is formed, in the single member, with a laser having a pulse duration of a picosecond order or less, and removing the first modified region, the second modified region, and the third modified region from the single member by etching.
A method for manufacturing a base body according to a third aspect of the present invention includes: a process B 1 of preparing a base member including a single member; forming a channel and a slow flow portion in the single member; a process B2 of forming a modified region by irradiating a region in which a fine vacuum hole is formed, in the single member, with a laser having a pulse duration of a picosecond order or less, and a process B3 of removing the modified region from the single member by etching.
A method for manufacturing a base body according to a fourth aspect of the present invention includes: a process C1 of preparing a base member including a single member; forming a modified region by irradiating, at least, a region in which a fine vacuum hole is formed and a portion of a region in which a channel is formed, in the single member, with a laser having a pulse duration of a picosecond order or less, a process C2 of forming the channel and the slow flow portion in the single member, and a process C3 of removing the modified region from the single member by etching.
According to a fifth aspect of the present invention, there is provided a method for manufacturing a base body, including: a process D1 of preparing a base member including a single member; forming a modified region by irradiating, at least, a region in which a fine vacuum hole is formed and a region in which a slow flow portion is formed, in the single member, with a laser having a pulse duration of a picosecond order or less, a process D2 of forming the channel and the slow flow portion in the single member, and a process D3 of removing the modified region from the single member by etching.

### EFFECTS OF THE INVENTION

According to the base body of the aspects of the present invention, it is possible to adsorb and trap a fine particle in the adsorption portion formed on a first end of a fine vacuum hole which opens on the channel into which a fluid including the fine particle flows, by suctioning the fine vacuum hole from the outside of a base member. Since the adsorption portion is formed of a single member, it is possible to substantially eliminate a step without a joint. For this reason, the trapped fine particle is caused to adhere sufficiently closely to the adsorption portion, and thus it is possible to stably maintain a state where the fine particle is trapped. Therefore, the fine particle is easily observed. Further, when the fine particle is a microorganism or a cell, it is possible to perform a high-accuracy electrophysiological measurement.
In addition, a slow flow portion is provided at a position close to the adsorption portion, and thus it is possible to slow the flow of the fluid at a position close to the adsorption portion. As a result, since the fine particle can be caused to remain at a position close to the adsorption portion, the fine particle is easily trapped in the adsorption portion. In addition, since pressure which is applied to the fine particle, after becoming trapped from a fluid decreases, the trapped fine particle can be trapped stably and continuously without being stripped off by a fluid.

In the channel, when the slow flow portion is provided in the inner wall surface different from the adsorption portion, it is possible to add another function to the slow flow portion, in addition to a function of slowing the flow of a fluid. For example, when a slow flow portion having a recess portion is provided at the bottom of the channel, the recess portion can slow the flow of a fluid, and precipitate and trap a fine particle in the recess portion. The fine particle trapped in the slow flow portion can be suctioned in the adsorption portion provided at a position close to the slow flow portion, and be trapped in the adsorption portion. That is, it is possible to obtain an effect that the trapping of the fine particle by the slow flow portion makes it easier for the fine particle to be suctioned and adsorbed in the adsorption portion.
In this manner, when the slow flow portion is provided in the inner wall surface different from the adsorption portion, and the adsorption portion is provided at one lateral side of the channel, there are, for example, the bottom of the channel, the other lateral side, and the ceiling surface, as options of the surface on which the slow flow portion is formed. That is, since the options of the surface on which the slow flow portion is formed increase, the design freedom of the channel and the slow flow portion increases.

In the channel, when the slow flow portion is provided in the inner wall surface which is flush with the adsorption portion, the slow flow portion and the adsorption portion can be formed integrally with each other. For example, it is possible to provide a slow flow portion having a recess portion at the lateral side of the channel, and to form the adsorption portion in the inner surface of the recess portion. In this case, since a main stream of a fluid flowing through the channel can further reduce the extent of being sprayed on the adsorption portion directly, a disadvantage in that the fine particle once adsorbed is stripped off from the adsorption portion by the main stream is almost eliminated, and thus it is possible to trap the fine particle stably and continuously.

According to a method for manufacturing a base body of the aspects of the present invention, it is possible to form a fine vacuum hole in a single member, and to cause a first end of the fine vacuum hole to communicate with a channel provided within the base body. When the method for forming the fine vacuum hole is a method for forming a modified region in a base member by laser irradiation, and removing the modified region using an etching process, it is possible to create a base body in which the fine vacuum hole is disposed in the base member accurately and in a high density. Specifically, it is possible to form the adsorption portion with a diameter of the opening on the order of nanometers, the adsorption portion being included in the first end of the fine vacuum hole.

### BRIEF DESCRIPTION OF THE DRAWINGS

FIG. 1 is a schematic perspective view illustrating an example of a base body according to the present invention.
FIG. 2 is a cross-sectional view taken along line A-A of FIG. 1.
FIG. 3 is a schematic diagram illustrating a state where a fine particle is trapped in an adsorption portion in the cross-sectional view of FIG. 2.
FIG. 4 is a schematic perspective view illustrating chief parts in the vicinity of a slow flow portion P in an example of the base body.
FIG. 5 is a schematic perspective view illustrating chief parts in the vicinity of the slow flow portion P in an example of the base body.
FIG. 6 is a schematic perspective view illustrating chief parts in the vicinity of the slow flow portion P in an example of the base body.
FIG. 7 is a schematic perspective view illustrating an example of the base body according to the present invention.
FIG. 8 is a schematic perspective view illustrating an example of the base body according to the present invention.
FIG. 9 is a schematic perspective view illustrating an example of the base body according to the present invention.
FIG. 10 is a schematic perspective view illustrating an example of the base body according to the present invention.
FIG. 11 is a schematic perspective view illustrating an example of the base body according to the present invention.
FIG. 12 is a cross-sectional view taken along line A-A of FIG. 11.
FIG. 13 is a schematic diagram illustrating a state where the fine particle is trapped in the adsorption portion in the cross-sectional view of FIG. 12.
FIG. 14 is a schematic perspective view illustrating an example of the base body according to the present invention.
FIG. 15 is a cross-sectional view taken along line A-A of FIG. 14.
FIG. 16 is a schematic diagram illustrating a state where the fine particle is trapped in the adsorption portion in the cross-sectional view of FIG. 15.
FIG. 17 is a cross-sectional view illustrating an example of the base body according to the present invention.
FIG. 18 is a cross-sectional view illustrating an example of the base body according to the present invention.
FIG. 19 is a schematic perspective view illustrating an example of the base body according to the present invention.
FIG. 20 is a cross-sectional view taken along line A-A of FIG. 19.
FIG. 21 is a schematic top view illustrating an example of the base body according to the present invention.
FIG. 22 is a schematic top view illustrating an example of the base body according to the present invention.
FIG. 23 is a schematic perspective view illustrating an example of the base body according to the present invention.
FIG. 24 is a cross-sectional view taken along line A-A of FIG. 23.
FIG. 25 is a schematic diagram illustrating a state where the fine particle is trapped in the adsorption portion in the cross-sectional view of FIG. 24.
FIG. 26 is a schematic perspective view illustrating chief parts in the vicinity of the slow flow portion P in an example of the base body.
FIG. 27 is a schematic diagram illustrating a longitudinal cross-section of a fine vacuum hole in an example of the base body.
FIG. 28 is a schematic diagram illustrating a longitudinal cross-section of the fine vacuum hole in an example of the base body.
FIG. 29 is a schematic diagram illustrating a longitudinal cross-section of the fine vacuum hole in an example of the base body.
FIG. 30 is a schematic diagram illustrating a longitudinal cross-section of the fine vacuum hole in an example of the base body.
FIG. 31 is a schematic perspective view illustrating an example of the base body according to the present invention.
FIG. 32 is a schematic diagram illustrating a longitudinal cross-section of the fine vacuum hole in an example of the base body.
FIG. 33 is a schematic perspective view illustrating an example of the base body according to the present invention.
FIG. 34 is a schematic diagram illustrating a longitudinal cross-section of the fine vacuum hole in an example of the base body.
FIG. 35 is a schematic perspective view illustrating an example of the base body according to the present invention.
FIG. 36 is a schematic diagram illustrating a longitudinal cross-section of the fine vacuum hole in an example of the base body.
FIG. 37 is a schematic diagram illustrating a longitudinal cross-section of the fine vacuum hole in an example of the base body.
FIG. 38 is a schematic perspective view illustrating an example of the base body according to the present invention.
FIG. 39 is a schematic diagram illustrating a longitudinal cross-section of the fine vacuum hole in an example of the base body.
FIG. 40 is a schematic top view illustrating an example of the base body according to the present invention.
FIG. 41 is a schematic top view illustrating an example of the base body according to the present invention.
FIG. 42A is a schematic cross-sectional view illustrating an example of a method for manufacturing the base body according to the present invention.
FIG. 42B is a schematic cross-sectional view illustrating an example of a method for manufacturing the base body according to the present invention.
FIG. 42C is a schematic cross-sectional view illustrating an example of a method for manufacturing the base body according to the present invention.
FIG. 42D is a schematic cross-sectional view illustrating an example of a method for manufacturing the base body according to the present invention.
FIG. 43 is a schematic perspective view illustrating a laser irradiation method α.
FIG. 44A is a diagram schematically illustrating a relationship between laser irradiation energy and a formed modified region (low oxygen concentration portion).
FIG. 44B is a diagram schematically illustrating a relationship between the laser irradiation energy and the formed modified region (low oxygen concentration portion).
FIG. 44C is a diagram schematically illustrating a relationship between the laser irradiation energy and the formed modified region (low oxygen concentration portion).
FIG. 44D is a diagram schematically illustrating a relationship between the laser irradiation energy and the formed modified region (low oxygen concentration portion).
FIG. 45A is a schematic cross-sectional view illustrating an example of a method for manufacturing the base body according to the present invention.
FIG. 45B is a schematic cross-sectional view illustrating an example of a method for manufacturing the base body according to the present invention.
FIG. 45C is a schematic cross-sectional view illustrating an example of a method for manufacturing the base body according to the present invention.
FIG. 45D is a schematic cross-sectional view illustrating an example of a method for manufacturing the base body according to the present invention.
FIG. 46A is a schematic cross-sectional view illustrating an example of a method for manufacturing the base body according to the present invention.
FIG. 46B is a schematic cross-sectional view illustrating an example of a method for manufacturing the base body according to the present invention.
FIG. 46C is a schematic cross-sectional view illustrating an example of a method for manufacturing the base body according to the present invention.
FIG. 47A is a schematic cross-sectional view illustrating an example of a method for manufacturing the base body according to the present invention.
FIG. 47B is a schematic cross-sectional view illustrating an example of a method for manufacturing the base body according to the present invention.
FIG. 47C is a schematic cross-sectional view illustrating an example of a method for manufacturing the base body according to the present invention.
FIG. 47D is a schematic cross-sectional view illustrating an example of a method for manufacturing the base body according to the present invention.
FIG. 48A is a schematic cross-sectional view illustrating an example of a method for manufacturing the base body according to the present invention.
FIG. 48B is a schematic cross-sectional view illustrating an example of a method for manufacturing the base body according to the present invention.
FIG. 48C is a schematic cross-sectional view illustrating an example of a method for manufacturing the base body according to the present invention.
FIG. 48D is a schematic cross-sectional view illustrating an example of a method for manufacturing the base body according to the present invention.
FIG. 49 is a schematic cross-sectional view illustrating an example of a device configuration used in a patch-clamp method in the related art.

### EMBODIMENTS FOR CARRYING OUT THE INVENTION

Hereinafter, the present invention will be described with reference to the accompanying drawings on the basis of preferred embodiments.

### <First Embodiment of Base Body>

### [Base Body 10A]

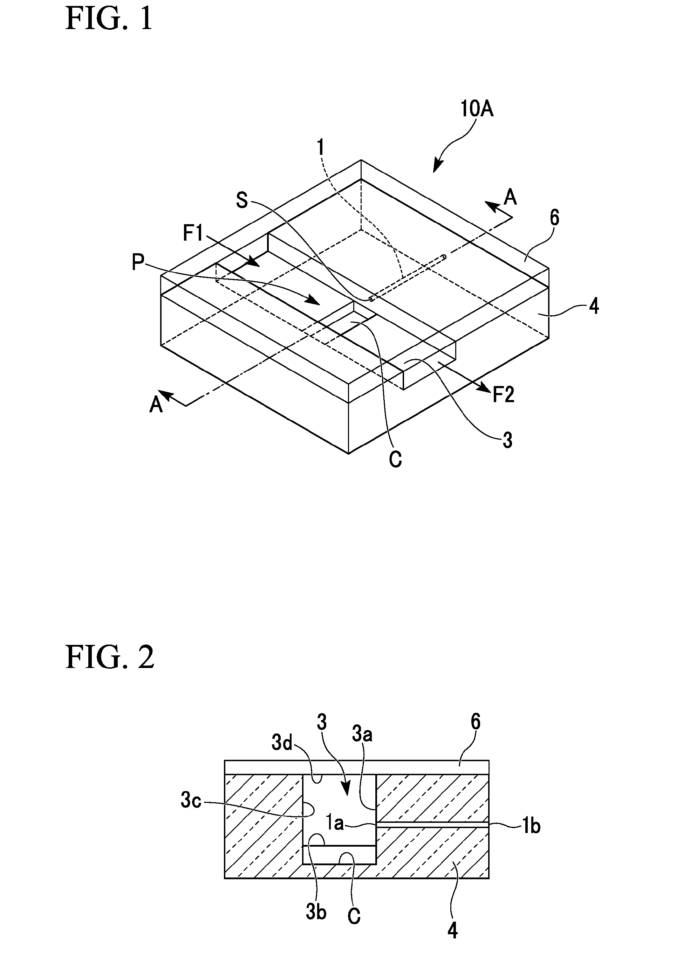

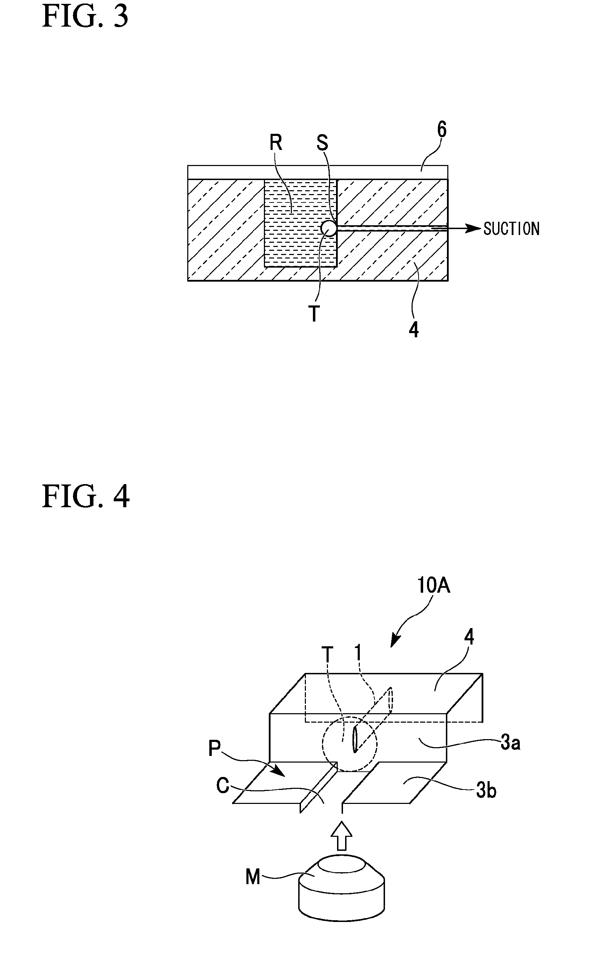

FIG. 1 is a perspective view illustrating a base body 10A which is a first embodiment of a base body according to the present invention. FIGS. 2 and 3 are schematic diagrams illustrating a cross-section taken along line A-A of FIG. 1.

The base body 10A is a base body including a fine vacuum hole 1 that traps a fine particle T. The base body 10A includes a channel 3, provided within a base member 4, which circulates a fluid R including the fine particle T, and the fine vacuum hole 1 that communicates between the channel 3 and the outside of the base member 4, and at least a portion that configures the fine vacuum hole 1 in the base member 4 is formed of a single member. Further, a substrate 6 is bonded so as to cover the upper surface of the base member 4. When an observation is performed through the base member 6, the base member 6 preferably transmits visible light.
In addition, in the base body 10A, a slow flow portion P that slows the flow of the fluid R is disposed at a position close to a first opening (opening) of the fine vacuum hole 1 in an inner wall constituting the channel 3.

In the base body 10A, the channel 3 is provided on the upper surface side of the base member 4. In the channel 3, the fluid R including the fine particle T is circulated from the upstream side F1 to the downstream side F2.
An adsorption portion S on which a first opening 1a of the fine vacuum hole 1 is exposed is formed at the lateral side 3a of the channel 3, and at least a portion of one of at least upper the surface 3d and lower surface 3b of the channel 3 is constituted by the transparent member 6 (substrate 6) so as to optically observe the fine particle T trapped in the adsorption portion S.

FIG. 3 is a diagram illustrating a state where the fluid R is circulated through the channel 3 of the base body 10A, and the fine particle T contained in the fluid R is trapped in the adsorption portion S.

The fine particle T trapped by the base body of the present invention is contained in the fluid R, and is not particularly limited insofar as it can circulate through the channel 3. For example, the fine particle may include particles formed of a microorganism, a cell, and an organic substance, particles formed of an inorganic substance, and the like. The microorganism may include bacteria, fungi, molds, large-size viruses, and the like. The cell may include cells capable of suspension culture of erythrocytes, leukocytes, and the like. The particle formed of an organic substance may include particles constituted by macromolecules such as a resin or polysaccharide, active carbon particles, and the like. The particle formed of an inorganic substance may include metal particles such as a silica particle or a gold colloid particle.
The particle formed of an organic substance and the particle formed of an inorganic substance may be a functional particle in which antibody molecules or the like is bonded to the surface or inside thereof.
The shape of the particle formed of an organic substance and the shape of the particle formed of an inorganic substance are not particularly limited. For example, every particle having a three-dimensional shape such as a circle, a cube, a cuboid, a polyhedron, a doughnut-shaped solid, or a string-shaped solid is included in the fine particle of the present invention.
The size of the particle formed of an organic substance and the size of the particle formed of an inorganic substance may preferably be larger than the opening diameter (minor diameter) of a first end of the fine vacuum hole having the adsorption portion, and are not particularly limited. That is, the size may preferably be as large as the particles do not pass through the fine vacuum hole.

As the fine particle trapped by the base body of the present invention, a microorganism or a cell is preferable. The surface shape of the microorganism or the cell is relatively flexible. For this reason, the microorganism or the cell adheres sufficiently closely to the adsorption portion S, and thus can be adsorbed therein more strongly. Furthermore, when the fine particle is a microorganism or a cell, it is possible to perform a high-accuracy electrophysiological measurement.

As shown in FIG. 3, when an observation from the lower surface side of the base member 4 or the upper surface side of the base member 4 is performed in a case where the fine vacuum hole 1 opens on the lateral side 3 a of the channel 3, the fine vacuum hole 1 and an object to be observed do not overlap each other, and thus the fine particle T can be easily observed.
FIG. 4 is a diagram illustrating a state where the fine particle T is optically observed while an objective lens M of a microscope moves closer to the lower surface of the base member 4. Meanwhile, in FIG. 4, only chief parts in the vicinity of the slow flow portion P in the base body 10A are shown.

When the trapped fine particle is observed from the lower surface of the base member, the distance between (working distance) an object to be observed and an objective lens is adjusted and the object to be observed is brought into focus. However, when the distance between the lower surface of the base member and the object to be observed is larger than the working distance required to be observed, the object to be observed cannot be brought into focus. In order to avoid such a situation, the thickness of the base member is reduced by polishing the lower surface of the base member, and thus it is possible to shorten the distance between the lower surface of the base member and the object to be observed. However, when the fine vacuum hole that opens on the lower surface 3b of the channel 3 is formed, the fine vacuum hole is present between the lower surface 3b of the channel 3 and the lower surface of the base member, and thus it may be difficult to reduce the thickness of the base member by polishing the lower surface of the base member. Therefore, as in the base body 10A, the fine vacuum hole preferably opens on the lateral side 3 a of the channel 3.

In the present invention, the fluid R including the fine particle T which is an object to be observed is not particularly limited, and includes, for example, blood, cell culture liquid, liquid for drink, river water, and the like. In addition, air including molds and the like is also included in the fluid R.

In the present invention, "circulation of the fluid R through the channel 3" means that the fluid R is put into the channel 3 from the outside of the base body 10A and is allowed to flow therethrough. The fluid R flowing into the channel may be continuously circulated through the channel 3, and the circulation thereof may be temporarily stopped, as necessary. The fluid R flowing through the channel 3 is finally discharged to the outside of the base body 10A, the same fluid R may be allowed to flow into the channel 3 again, as necessary, and may be circulated. This is applied to all the base bodies according to the present invention.

In the channel 3 of the base body 10A, a first inner wall surface 3b (lower surface 3b) on which the slow flow portion P is disposed and a second inner wall surface 3a (lateral side 3a) on which the first opening 1a of the fine vacuum hole 1 is disposed are inner wall surfaces different from each other. That is, the lower surface 3b on which a recess portion C included in the slow flow portion P is provided and the lateral side 3 a on which the first end 1a of the fine vacuum hole 1 opens are formed on the inner wall surfaces different from each other in the channel 3.

The slow flow portion P provided at a position close to the adsorption portion S has the recess portion C disposed immediately below the adsorption portion S, in the lower surface (bottom) 3b of the channel 3.
Turbulent flows including components such as, for example, an upward flow, a downward flow, a backflow, and an eddy flow occur in the flow of the fluid R at the inside of the recess portion C or the position close to the recess portion C in the slow flow portion P in which the recess portion C is disposed. That is, since the fluid R flows nonlinearly at the inside of the recess portion C or the position close to the recess portion C, the flow of the fluid R becomes slow. The fine particle T in the fluid R is involved in the turbulent flow at the time of passing through the position close to the slow flow portion P. Therefore, in the channel of the present invention, the time for which the fine particle T stagnates at the position close to the slow flow portion P can be made longer than in a channel which is not provided with the slow flow portion P. As a result, the adsorption portion S provided at the position close to the slow flow portion P can easily trap the fine particle T.

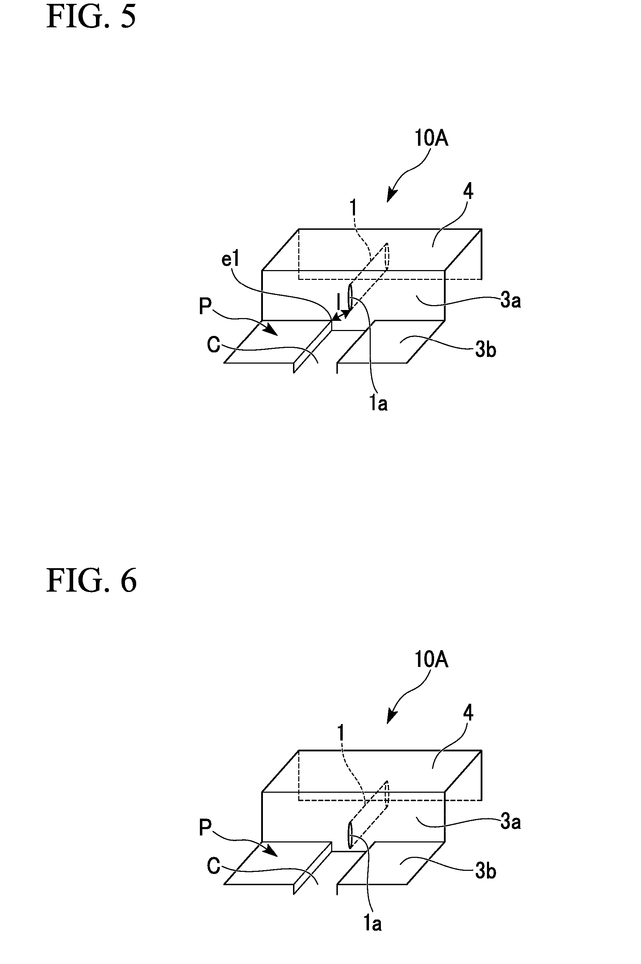

In the slow flow portion P of the present invention, the shortest distance 1 between the first opening 1a of the fine vacuum hole 1 and the end e1, at which an opening is formed, in the corner included in the recess portion C on the side close to the first opening 1a is preferably 0 µm to 500 µm, more preferably 0.001 µm to 250 µm, and still more preferably 0.01 µm to 100 µm (see FIG. 5).
When the shortest distance 1 is in the above-mentioned range, the adsorption portion S is provided within the sphere of the influence of the turbulent flow generated in the slow flow portion P, the adsorption portion S can more easily trap the fine particles. At this time, the fluid R may flow in any of the directions.
Also, in FIG. 5, only chief parts in the vicinity of the slow flow portion P in the base body 10A are shown.

In the channel 3, the recess portion C may be provided in any of the surfaces of the lateral sides 3a and 3b, the lower surface 3b, or the upper surface 3d. When the recess portion C is disposed in the lower surface 3b, the fine particle T may be temporarily caught within the recess portion C and be held therein. The fine particle T temporarily held has a tendency to be suctioned and trapped in the first opening 1a disposed at the position close to the recess portion C. For this reason, the recess portion C is preferably disposed in the lower surface 3b of the channel 3.

The height at which the first opening 1a is disposed in the fine vacuum hole 1 may not be included in the recess portion C as shown in FIG. 5, and may be included in the recess portion C as shown in FIG. 6.
When at least a portion of the first opening 1a is included in the recess portion C, the adsorption portion S is configured to be disposed within the recess portion C. In this case, this is preferable because the fine particle T temporarily held within the recess portion C can be pulled toward the adsorption portion S more reliably and be trapped therein.

In the slow flow portion P of the present invention, the depth of the recess portion C is preferably in a range of 0.5 µm to 50 µm, more preferably in a range of 1 µm to 25 µm, and still more preferably in a range of 1 µm to 10 µm. When the depth is in the above-mentioned range, it is possible to generate the turbulent flow sufficiently in the slow flow portion P.
The width of the recess portion C is preferably in a range of 0.5 µm to 100 µm, more preferably in a range of 1 µm to 50 µm, and still more preferably in a range of 5 µm to 25 µm. When the width is in the above-mentioned range, it is possible to generate the turbulent flow sufficiently in the slow flow portion P.

In the base body 10A shown in FIG. 1, it is preferable that the single member not only include the fine vacuum hole 1, but also constitute the entirety of the base member 4.
A material of the single member includes, for example, silicon, glass, silica, sapphire, or the like. Above all, the material is preferably an amorphous material which is not likely to be influenced by processing anisotropy due to a crystal orientation. When such a material is used, the processing accuracy of the fine vacuum hole 1 can be further increased, and the adsorption portion S is preferably formed with a diameter of the opening on the order of nanometers. For this reason, it is relatively easy to form a high-resistance seal between the trapped cell T (fine particle T) and the adsorption portion S. At this time, when the adsorption portion S formed in the fine vacuum hole 1 is formed of glass, silica, or sapphire, this is preferable because a process of providing an insulation quality is not required. When a high-resistance seal is formed, and an electrophysiological measurement of the trapped cell T is performed, a method of measuring the potential difference (membrane potential) or the like between the inside and outside of the cell T can also be appropriately used in the base body to perform the measurement. When a material of which the adsorption portion S is formed is glass or silica, and the electrophysiological measurement of the trapped cell T is performed, a high-resistance seal on the order of gigaohms between the cell T and the adsorption portion S is realized, and thus a higher-accuracy measurement can be made.

The material of the single member preferably transmits light having at least a portion of a wavelength in light having a wavelength of 0.1 µm to 10 µm.
Specifically, it is preferable to transmit at least a portion of normal light (wavelength of 0.1 µm to 10 µm) used as a processing laser. It is possible to form modified regions by irradiating, with such a laser, to the member as described below.
In addition, the material of the single member more preferably transmits light of a visible light region (wavelength of approximately 0.36 µm to approximately 0.83 µm). The light of a visible light region is transmitted, thereby allowing the trapped fine particle T to be easily observed through the single member using an optical method of an optical microscope or the like.
Furthermore, when the material of the single member transmits at least a portion of ultraviolet light or visible light (0.1 µm to 0.83 µm) in the light of the wavelength, it is possible to perform a fluorescent observation on a tissue within a microorganism or a cell (fine particle T) dyed by a fluorescent dye.
Meanwhile, "transmissive (transparent)" in the present invention refers to all the states where light is caused to be incident on the member, and transmitted light is obtained from the member.
In FIG. 1, the single member constituting the base member 4 is a transparent glass substrate.

As shown in FIGS. 2 and 3, the fine vacuum hole 1 communicates between the channel 3 and the outside of the base member 4. The first end 1a of the fine vacuum hole 1 is exposed (opens) onto the lateral side 3a of the channel 3, and constitutes the adsorption portion S. The second end 1b of the fine vacuum hole 1 is exposed onto the lateral side of the base member 4.

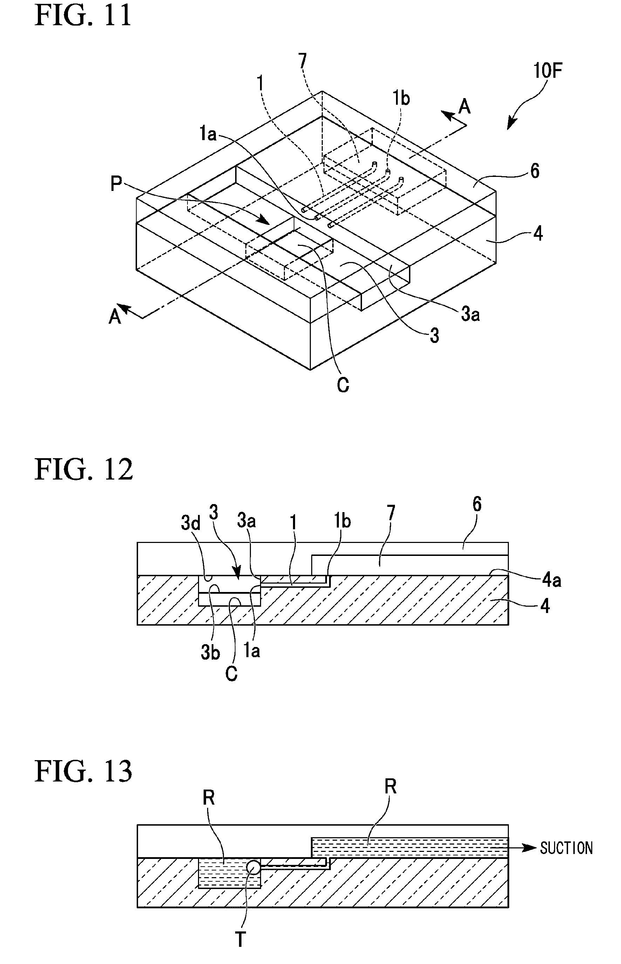

In the present invention, when the fine vacuum hole 1 communicates between the channel 3 and the outside of the base member 4, the second opening 1b of the fine vacuum hole 1 may be exposed onto the lateral side (outside) of the base member 4 as in the base body 10A of FIG. 1.
Further, for example, as in the fine vacuum hole 1 in a base body 10F (FIG. 11) described later, the second opening 1b of the fine vacuum hole 1 may open onto a second channel 7, and the fine vacuum hole 1 may communicate with the outside of the base member 4 through the second channel 7.
The meaning of "the fine vacuum hole 1 communicates between the channel 3 and the outside of the base member 4" mentioned above is applied to all the base bodies according to the present invention.

The fluid R within the channel 3 is suctioned from the second opening 1b side of the fine vacuum hole 1 which is exposed to the outside of the base member 4, thereby allowing the fine particle T contained in the fluid R to be trapped in the adsorption portion S. A dynamic force of the suction is not particularly limited, but the fluid can be suctioned by connecting a suction mechanism (not shown) such as, for example, a syringe or a pump to the second opening 1b of the fine vacuum hole 1.

In the base body 10A, the fine vacuum hole 1 is formed in the single glass substrate 4 (base member 4), and is a through hole which does not have a joint or a bonded surface. As a matter of course, a joint or a bonded surface is also not present in the adsorption portion S on the end of the fine vacuum hole 1. For this reason, it is possible to sufficiently increase adhesion between the fine particle T and the adsorption portion S. In addition, when an external force such as the deformation of the glass substrate 4 and erosion due to a chemical solution is applied, a bonded surface is not present in the fine vacuum hole 1 or in the vicinity of the fine vacuum hole 1, peeling or damage in the bonded surface does not occur. Thus, even when heating sterilization or chemical solution sterilization is repeatedly performed on the glass substrate 4, the glass substrate 4 is not damaged. This is a particularly excellent characteristic of the base body to routinely perform heating sterilization or chemical solution sterilization, and to trap a microorganism or a cell (fine particle T). Further, since refractive index difference does not occur in the vicinity of the fine vacuum hole 1, light from the adsorption portion S is more likely to be condensed. Therefore, it is possible to easily observe the trapped fine particle. The term "adsorption portion S" as used herein refers to a region that comes into contact with or comes close to the fine particle T in the lateral side 3 a of the channel 3.

A hole, having the adsorption portion S, of the first opening 1a of the fine vacuum hole 1 in the lateral side 3a of the channel 3 may have any of the shapes. For example, the hole can be circular, substantially circular, elliptic, substantially elliptic, rectangular, triangular, or the like. When the shape of the hole is circular or substantially circular, the diameter thereof may preferably be in a range of 0.02 µm to 5 µm. Above all, the diameter of a microorganism (fine particle T) having a smaller size than that of a cell is more preferably in a range of 0.02 µm to 0.8 µm. When the shape is elliptic or substantially elliptic, and the minor diameter (shortest diameter) of the hole is in a range of 0.02 µm to 5 µm, the microorganism or the cell T (fine particle T) can be trapped. That is, the minor diameter of the hole may preferably be set to such a size that the fine particle T cannot pass through the fine vacuum hole 1. For example, when an erythrocyte cell (6 to 8 µm) is trapped, the minor diameter may preferably be approximately 1 µm. When bacillus natto (bacillus subtilis; 0.7 µm to 2 µm) is trapped, the minor diameter may preferably be approximately 0.2 µm.

The range of the minor diameter is preferably 0.02 µm to 2 µm. Above all, in order to trap a microorganism (fine particle T) having a smaller size than that of a cell, the diameter thereof is more preferably in a range of 0.02 µm to 0.8 µm.
When the diameter is less than a lower limit of the above-mentioned range, a suction force of the adsorption portion S is too weak, and thus there is a concern that the fine particle T is not able to be trapped. When the diameter exceeds an upper limit of the above-mentioned range, the fine particle T passes through the fine vacuum hole 1, and thus there is a concern that the fine particle is not able to be trapped.
On the other hand, the major diameter (longest diameter) of the hole may preferably be appropriately adjusted depending on the size of the fine particle T to be trapped, and includes, for example, a range of 0.2 µm to 10 µm. Further, when an electrophysiological measurement is performed, the major diameter is preferably made smaller than the sizes of a cell and a microorganism, and includes, for example, a range of 0.2 µm to 5 µm.

Further, when the short side of the diameter of the hole is highly accurately processed on the order of nanometers (nm unit), it is possible to trap a microorganism (fine particle) having a smaller size than that of a cell. Therefore, in the related art, microorganisms observed as a group can be observed singly or only a few. Thereby, various biological characteristics of microorganisms which have not elucidated so far can be confirmed. Further, by making the diameter of the adsorption portion of the hole very small, stable sealing between the cell membrane and the adsorption portion is realized at the time of measuring the electrophysiological characteristics of a cell, and thus the electrophysiological characteristics thereof can be stably measured.

In FIGS. 2 and 3, the fine vacuum hole 1 is formed so as to be substantially perpendicular to the lateral side 3 a of the channel 3. However, the fine vacuum hole is not necessarily required to be substantially perpendicular thereto, but can be freely disposed in the single glass substrate 4 in accordance with the design of the base body 10A.
Further, the diameter at a position close to the first opening 1a of the fine vacuum hole 1 can also be made slightly larger than the diameter of the fine vacuum hole 1. At this time, since a portion of the fine particle T can be trapped in a state where it enters the first opening 1a of the fine vacuum hole 1, more stable trapping for a long period of time may be able to be performed even when a flow is present in the channel 3.
A plurality of fine vacuum holes 1 may be disposed in the base body 10A. Since each of the fine vacuum holes 1 is provided with the adsorption portion S, it is possible to trap a plurality of fine particles T.

In the base body 10A, the lower surface 3b of the channel 3 is provided in the base member 4 formed of a glass substrate. On the other hand, the upper surface of the channel 3 facing the lower surface 3b is covered with the substrate 6 formed of glass. The fine particle T trapped in the adsorption portion S can be observed from at least one of the lower surface 3b and the upper surface 3d using an optical observation mechanism such as a microscope. Meanwhile, the upper surface 3d is not necessarily required to be covered by a lid, but may have no lid in an opened state. Normally, since the fluid R is circulated within the channel 3 by applying a pressure, the upper surface 3d of the channel 3 is preferably covered with a lid.

A material constituting the upper surface 3d of the channel 3 is not particularly limited, and includes, for example, a resin substrate such as PDMS and PMMA, a silicon substrate, and a glass substrate. Particularly, when the material is a material that transmits visible light or ultraviolet light, this is preferable because the fine particle T can be easily observed. In addition, when the material of the substrate 6 is the same material as that of the base member 4, this is preferable because the base member 4 and the substrate 6 can be easily bonded to each other. In addition, the bonding may be performed using a known method.

When the periphery of the adsorption portion S in the channel 3 can be observed, it can be confirmed that the fine particle T is trapped in the adsorption portion S. In this case, for example, when the microorganism or the cell (fine particle T) is trapped, the suction force of the fine vacuum hole 1 is made strong, and after it is trapped, the suction force is made weak to an extent that the cell membrane of the microorganism or the cell T is not damaged, thereby allowing the suction force to be properly adjusted depending on the state of the fine particle T in the adsorption portion S.

When an electrophysiological measurement of the trapped microorganism or cell T is performed, for example, electrodes (not shown) may be disposed on the channel 3 and the second opening 1b side of the fine vacuum hole 1, respectively. Alternatively, an external electrode through an extracellular buffer, an intracellular fluid or the like may be used. Since the adsorption portion S is formed of the single glass substrate 4, a high-resistance seal can be formed on the cell membrane of the cell T. Therefore, a patch-clamp method known in the related art can be applied. At this time, the hole diameter of the first opening 1a of the fine vacuum hole 1 having the adsorption portion S is made smaller than the hole diameter (approximately 2 µm to 4 µm) of a patch pipette or the like in the related art, thereby allowing an electrophysiological measurement having higher accuracy than in the related art to be performed.

### [Base Body 10B]

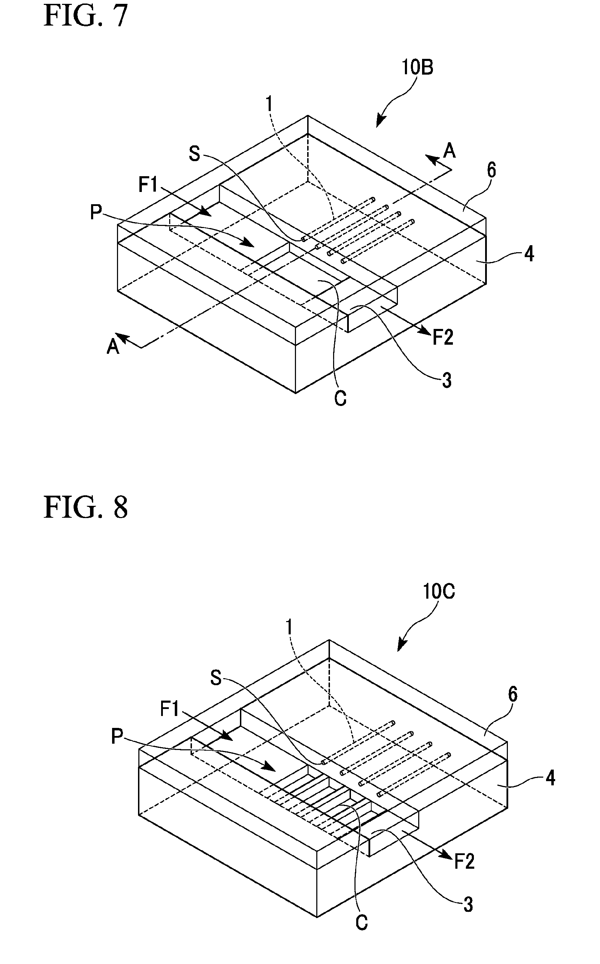

Another embodiment of the base body according to the present invention includes a base body 10B shown in FIG. 7. In FIG. 7, the same components as those of the above-mentioned base body 10A are denoted by the same reference numerals and signs. A cross-sectional view taken along line A-A of FIG. 7 is the same as those of FIGS. 2 and 3.
The base body 10B is different from the base body 10A in that a plurality of fine vacuum holes 1 are disposed parallel to each other. Other configurations of the base body 10B are the same as those of the base body 10A.
The first opening 1a of each of the fine vacuum holes 1 has the adsorption portion S. The recess portion C is disposed immediately below each of the adsorption portions S. The same effect as that of the base body 10A is also obtained in the base body 10B by the recess portion C.

When a plurality of fine vacuum holes 1 are disposed within the base member 4 as in the base body 10B, it is preferable that the gap between each of the fine vacuum holes 1 adjacent to each other be provided in consideration of the size of the trap fine particle T. For example, when the fine particle T having a size of approximately 3 µm is trapped, the gap between each of the fine vacuum holes 1 adjacent to each other is set to be equal to or more than 6 µm. As a result, since a plurality of trapped fine particles T do not come close to each other, it is possible to easily perform an observation and an electrophysiological measurement.

When a plurality of fine vacuum holes 1 open on the channel 3, a suction mechanism (not shown) such as an independent syringe or pump is connected to the second opening 1b of each of the fine vacuum holes 1, and thus the suction of each of the fine vacuum holes 1 can be individually controlled. Therefore, the trapping of the fine particle T by each of the fine vacuum holes 1 can be independently controlled.

Further, the hole diameters of the fine vacuum holes 1 may be the same as each other, or may be different from each other.
In addition, when the base member 4 is formed of silicon, glass, silica, sapphire or the like, the processing accuracy of the fine vacuum hole can be increased, thereby allowing a plurality of fine vacuum holes 1 to be densely disposed.

### [Base Body 10C]

Another embodiment of the base body according to the present invention includes a base body 10C shown in FIG. 8. In FIG. 8, the same components as those of the above-mentioned base body 10A are denoted by the same reference numerals and signs.
The base body 10C is different from the base body 10A in that a plurality of fine vacuum holes 1 are disposed parallel to each other, and that a plurality of recess portions C are disposed on the lower surface 3b of the channel 3. Other configurations of the base body 10C are the same as those of the base body 10A.
The first opening 1a of each of the fine vacuum holes 1 has the adsorption portion S. The recess portion C is disposed immediately below each of the adsorption portions S. The same effect as that of the base body 10A is also obtained in the base body 10C by the recess portion C. In addition, since a plurality of recess portions C are disposed, the extent of a turbulent flow having a possibility of occurring in the fluid R flowing along the vicinity of the slow flow portion P can be further increased than in the case of the base body 10B. That is, since the time for which the fine particle T is held in the vicinity of the slow flow portion P can be made longer, it is possible to easily trap the fine particle T in the adsorption portion S.

In the base body 10C, similarly to the base body 10B, it is also preferable that the gap between each of the fine vacuum holes 1 adjacent to each other be provided in consideration of the size of the fine particle T to be trapped. For example, when the fine particle T having a size of approximately 3 µ m is trapped, the gap between each of the fine vacuum holes 1 is set to be equal to or more than 6 µm. As a result, since a plurality of trapped fine particles T do not come close to each other, it is possible to easily perform an observation and an electrophysiological measurement.

Further, the hole diameters of the fine vacuum holes 1 may be the same as each other, or may be different from each other.
In addition, the sizes of recess portions C may be the same as each other, and may be different from each other.
Further, when the base member 4 is formed of silicon, glass, silica, sapphire or the like, the processing accuracy of the fine vacuum hole can be increased, thereby allowing a plurality of fine vacuum holes 1 and a plurality of recess portions C to be densely disposed.

### [Base Body 10D]

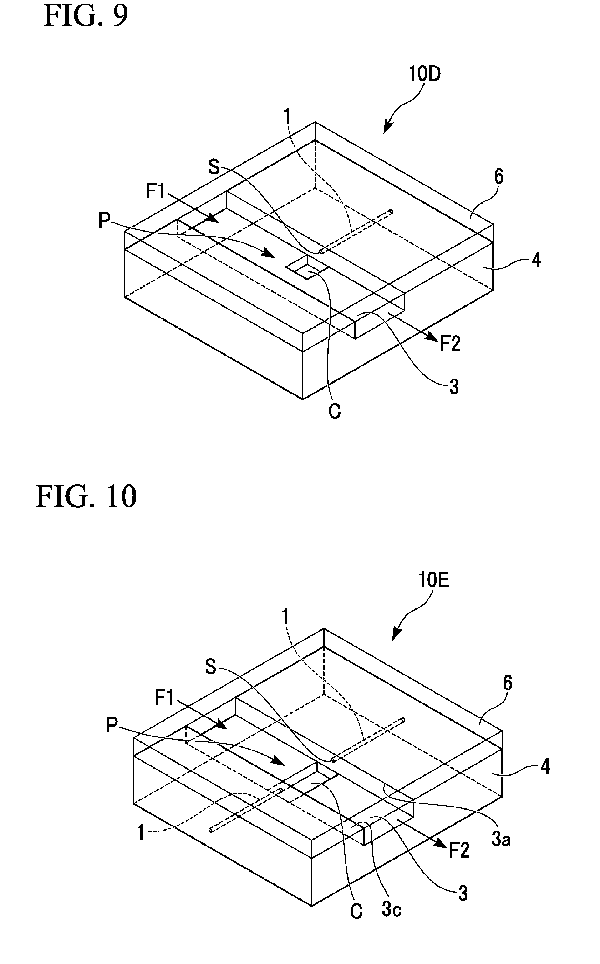

Another embodiment of the base body according to the present invention includes a base body 10D shown in FIG. 9. In FIG. 9, the same components as those of the above-mentioned base body 10A are denoted by the same reference numerals and signs.
The base body 10D is different from the base body 10A in that the length of the recess portion C along the extending direction of the fine vacuum hole 1 decreases by half, and that the capacity of the recess portion C decreases by half. Other configurations of the base body 10D are the same as those of the base body 10A.
The first opening 1a of the fine vacuum hole 1 has the adsorption portion S. The recess portion C is disposed immediately below the adsorption portion S. The same effect as that of the base body 10A is also obtained in the base body 10D by the recess portion C. However, the capacity of the recess portion C decreases by half, and thus the extent of a turbulent flow generated in the fluid R flowing along the inside of the recess portion C or the vicinity of the recess portion C becomes smaller than in the above-mentioned embodiment. For this reason, the extent that the flow of the fluid R in the slow flow portion P is slowed also becomes smaller than in the above-mentioned embodiment.

### [Base Body 10E]

Another embodiment of the base body according to the present invention includes a base body 10E shown in FIG. 10. In FIG. 10, the same components as those of the above-mentioned base body 10A are denoted by the same reference numerals and signs.
The base body 10E is different from the base body 10A in that in the channel 3, the first opening 1a of the fine vacuum hole 1 is disposed at both the lateral side 3 a and the lateral side 3c with the recess portion C interposed therebetween. Other configurations of the base body 10E are the same as those of the base body 10A.
The first opening 1a of each of the fine vacuum holes 1 has the adsorption portion S. A common recess portion C is disposed immediately below each of the adsorption portions S. The same effect as that of the base body 10A is also obtained in the base body 10B by the recess portion C.

### [Base Body 10F]

Another embodiment of the base body according to the present invention includes a base body 10F shown in FIG. 11. FIGS. 12 and 13 are schematic diagrams illustrating a cross-section taken along line A-A of FIG. 11. In FIGS. 11 to 13, the same components as those of the above-mentioned base body 10B are denoted by the same reference numerals and signs.
The base body 10F is different from the base body 10B in that each of the first openings 1a of the fine vacuum holes 1 plurally disposed opens onto the lateral side 3a of the channel 3 (first channel 3), and each of the second openings 1b opens onto the upper surface 4a of the base member 4. In the substrate 6, the second channel 7 is provided at a position facing the second opening 1b. According to such a configuration, a suction mechanism is directly or indirectly connected to the second channel 7, thereby allowing the fluid R within the first channel 3 to be pulled and suctioned into the second channel 7 through the fine vacuum hole 1. At this time, the fine particle in the fluid R can be trapped in the adsorption portion S. Other configurations of the base body 10F are the same as those of the base body 10B.

### [Base Body 10G]

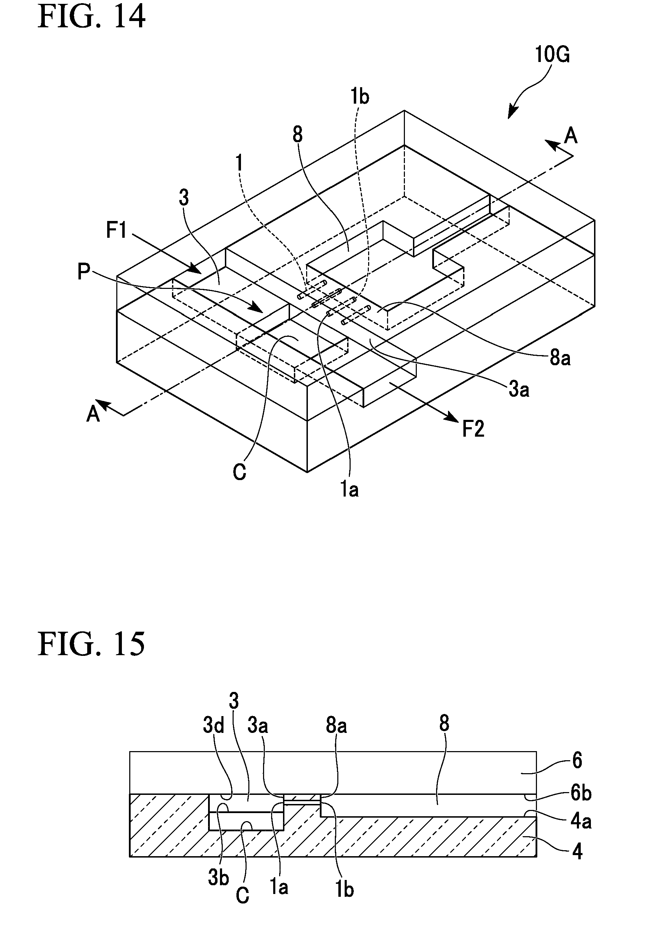

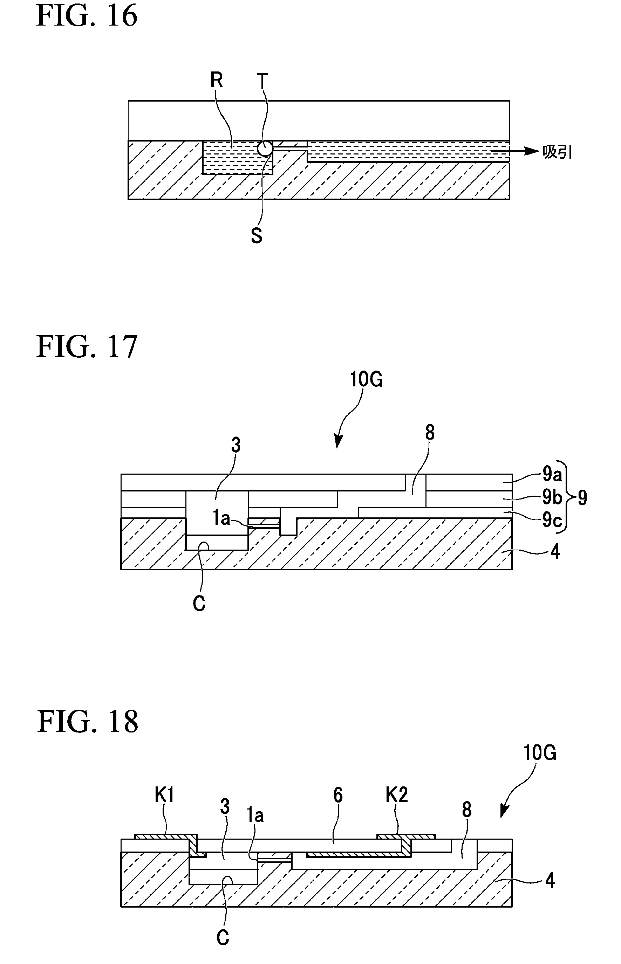

Another embodiment of the base body according to the present invention includes a base body 10G shown in FIG. 14. FIGS. 15 and 16 are schematic diagrams illustrating a cross-section taken along line A-A of FIG. 14. In FIGS. 14 to 16, the same components as those of the the above-mentioned base body 10B are denoted by the same reference numerals and signs. The base body 10G is different from the base body 10B in that each of the first openings 1a of the fine vacuum holes 1 plurally disposed opens onto the lateral side 3a of the channel 3 (first channel 3), and that each of the second openings 1b opens onto the lateral side 8a of a third channel 8. The first channel 3 and the third channel 8 which are disposed on the upper surface 4a of the base member 4 communicate with each other through the fine vacuum hole 1. Here, the first opening 1a and the second opening 1b of the fine vacuum hole 1 do not open to the outside of the base member 4 directly. However, since the third channel 8 communicates with the outside of the base member 4, the first channel 3 communicates with the outside of the base member 4 through the fine vacuum hole 1.
According to such a configuration, a suction mechanism is directly or indirectly connected to the third channel 8, thereby allowing the fluid R within the first channel 3 to be pulled and suctioned into the third channel 8 through the fine vacuum hole 1. At this time, the fine particle in the fluid R can be trapped in the adsorption portion S.
Other configurations of the base body 10G are the same as those of the base body 10B.

A modified example of the base body 10G includes a configuration in which another recess portion C is disposed immediately below the second opening 1b of the fine vacuum hole 1 in the lower surface 4a of the third channel 8. In a case of such a configuration, the fluid R is suctioned from the third channel 8 through the fine vacuum hole 1 to the first channel 3. The fine particle T contained in the fluid R flowing from the outside of the base member 4 into the third channel 8 can be adsorbed in the second opening 1b of the fine vacuum hole 1 by the suction. At this time, the recess portion C is disposed immediately below the second opening 1b, thereby allowing the fine particle T to be more easily trapped.
Therefore, another recess portion C is disposed in the inside of the third channel 8, and thus a method of causing the fluid R from the outside of the base member 4 into the third channel 8 can also be performed rather than a method of causing the fluid R to flow from the outside of the base member 4 into the first channel 3. That is, another recess portion C is disposed in the inside of the third channel 8, thereby allowing the convenience of the base body 10G to be enhanced.

In addition, a configuration in which the upper surface of the base member 4 is covered by a member 9 as shown in FIG. 17 can also be illustrated. In this case, a portion of the first channel 3 and a portion of the third channel 8 are constituted by the member 9. The thickness of the member 9 is adjusted, and the diameter of the first channel 3 is appropriately adjusted, thereby allowing the flow rate of the fluid R circulating through the first channel 3 to be appropriately controlled. In addition, when the member 9 is formed by bonding a plurality of members 9a, 9b, and 9c together, the third channel 8 can be provided within the member 9 on a desired path by changing a method of bonding each of the members 9a, 9b, and 9c together.
For example, it is possible to increase the diameter of the third channel 8 by laminating each of a plurality of members 9a, 9b, and 9c. Furthermore, using the height (thickness) of each of the laminated members 9a, 9b, and 9c, the side communicating with the outside of the third channel 8 can also be disposed on the upper surface of the base body 10G as shown in FIG. 17.

A material of the member 9 is not particularly limited, but a resin substrate such as PDMS and PMMA, or a glass substrate can be used. The member 9 may be formed of a material that transmits light (for example, ray of visible light) used for observation, and may be formed of a material that does not transmit light. When only the trapping of the fine particle in intended, it is not necessarily required to transmit light used for observation. A member that transmits light used for observation may be preferable because the observation can be performed optically from the upper surface.

When an electrophysiological measurement of the trapped microorganism or cell T is performed, electrodes K1 and K2 can be disposed in the first channel 3 and the third channel 8, respectively, for example, as shown in FIG. 18. Alternatively, it is possible to perform the electrophysiological measurement using an external electrode through an extracellular buffer, an intracellular fluid or the like. Since the adsorption portion S is formed of the single glass substrate 4 (base member 4), a high-resistance seal can be formed on the cell membrane of the cell T. Therefore, a patch-clamp method known in the related art can be applied. At this time, the hole diameter of the first opening 1a of the fine vacuum hole 1 having the adsorption portion S is made smaller than the hole diameter (approximately 2 µm to 4 µm) of a patch pipette or the like in the related art, thereby allowing an electrophysiological measurement having higher accuracy than in the related art to be performed.

Meanwhile, the electrodes K1 and K2 may be disposed other channels communicating with the first channel 3 and the third channel 8, for example, a fourth channel and a fifth channel which are described later.

### [Base Body 10H]

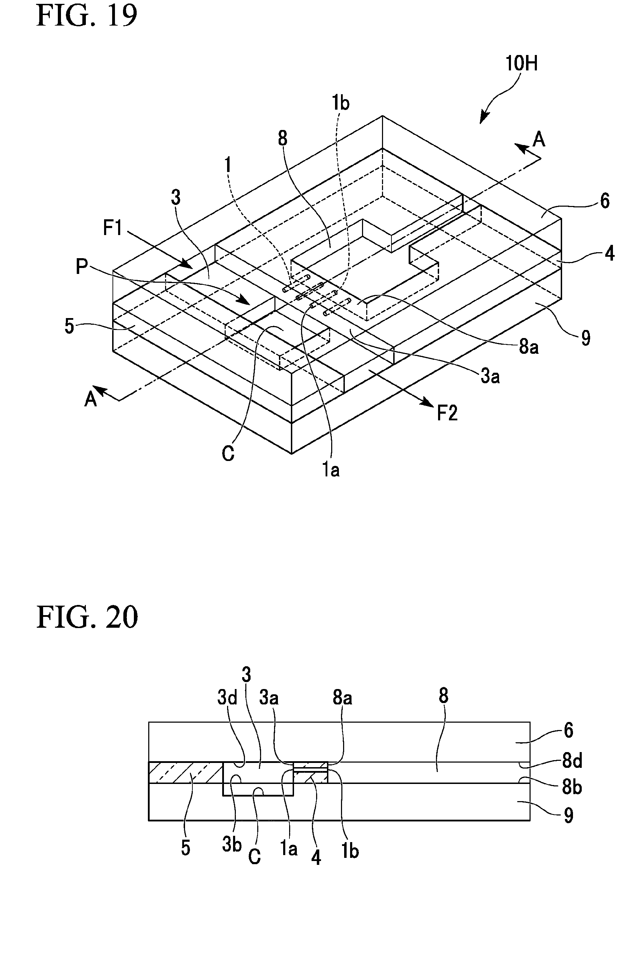

Another embodiment of the base body according to the present invention includes a base body 10H shown in FIG. 19. FIG. 20 is a schematic diagram illustrating a cross-section taken along line A-A of FIG. 19. In FIGS. 19 to 20, the same components as those of the above-mentioned base body 10G are denoted by the same reference numerals and signs.
The base body 10H is different from the base body 10G in that the lower surface 3b of the first channel 3 and the lower surface 8b of the third channel 8 are formed of the member 9. In this case, the fine vacuum hole 1 is also formed of the base member 4 constituted by a single material. A base member 5 interposed between the substrate 6 and the member 9 is formed of the same material as that of the base member 4. In addition, a material of the base member 5 may be the same material as that of the substrate 6 or the member 9. The recess portion C is formed on the upper surface of the member 9, and is disposed immediately below the adsorption portion S in the channel 3. Other configurations of the base body 10H are the same as those of the base body 10G.

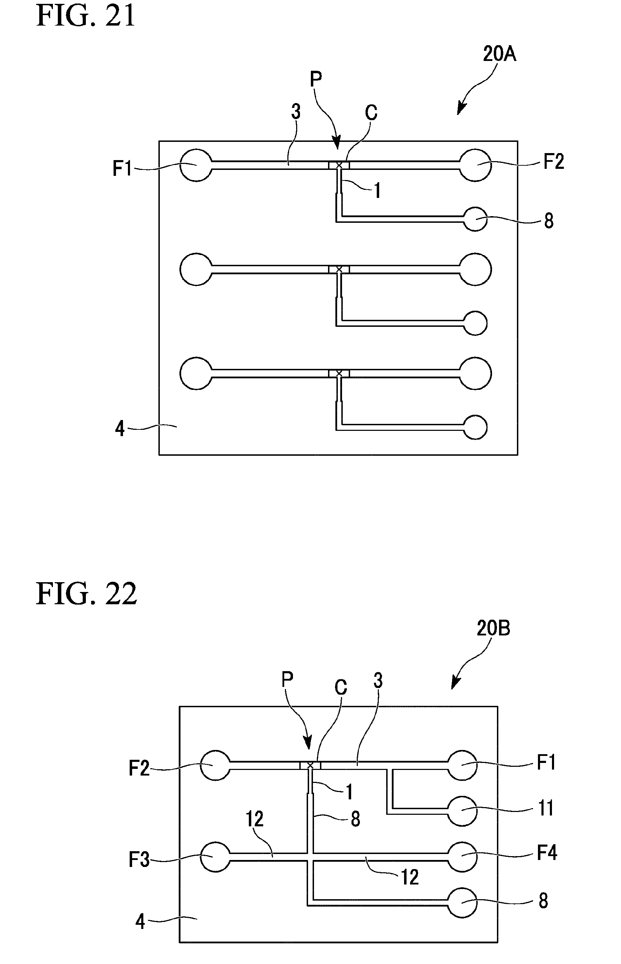

FIG. 21 is a schematic top view (perspective view seen from the upper surface) of a base body 20A which is an example of the base body according to the present invention. The arrangement configuration of the first channel 3, the fine vacuum hole 1, and the second channel 7 or the third channel 8 in the top view can be applied to the base body 10F to the base body 10H mentioned above.
Three sets of composite channels including the first channel 3 and the third channel 8 are disposed in the glass substrate 4.
Communication of the first channel 3 and the third channel 8 with each other through the fine vacuum hole 1 is as mentioned above. The position of the adsorption portion S is denoted by a sign of "X". The slow flow portion P having the recess portion C is provided at a position close to the adsorption portion S. The fluid R flows in from the upstream side F1 of the first channel 3, and is circulated to the downstream side F2 of the third channel 8.

In the base body 20A, a plurality of third channels 8 can also be disposed in the single first channel 3. At this time, at least one or more fine vacuum holes 1 are provided in the single third channel 8. In this manner, when a plurality of third channels 8 are disposed in the single first channel 3, a suction mechanism (not shown) such as a syringe or a pump is connected independently to each of the third channels 8, thereby allowing the suction of the third channels 8 to be controlled independently. Therefore, the trapping of the fine particle T by the fine vacuum hole 1 can be controlled independently.

FIG. 22 is a schematic top view (perspective view seen from the upper surface) of a base body 20B which is an example of the base body according to the present invention. The arrangement configuration of the first channel 3, the fine vacuum hole 1, the third channel 8, the fifth channel 12, and the fourth channel 11 in the top view can be applied to the base body 10F to the base body 10H mentioned above.

Communication of the first channel 3 and the third channel 8, disposed in the glass substrate 4, with each other through the fine vacuum hole 1 is as mentioned above. The position of the adsorption portion S is denoted by a sign of "X".
The fifth channel 12 disposed in the glass substrate 4 intersects the third channel 8 so as to communicate therewith. A desired chemical solution or gas is circulated from the upstream side F3 of the fifth channel 12 to the downstream side F4, thereby allowing the chemical solution or gas to be diffused and caused to flow into the third channel 8. In addition, the chemical solution diffused into the third channel 8 can also be replaced with a gas.
The place in which the fifth channel 12 communicates with the third channel 8 and the shape of the fifth channel 12 are not particularly limited. Therefore, for example, the fifth channel 12 can also be disposed at a position close to the fine vacuum hole 1, and each function of the fifth channel 12 and the third channel 8 can be substituted. In addition, the fifth channel 12 can also be configured to have only the F3 side or F4 side, and any function of F3 or F4 can also be fulfilled by the third channel 8. A plurality of fifth channels 12 or the fifth channels 12 branched into a plurality of parts may be disposed.

In addition, the fourth channel 11 disposed in the glass substrate 4 communicates with the first channel 3. A desired chemical solution or gas is circulated from the fourth channel 11 to the first channel 3, thereby allowing the chemical solution or gas to be diffused and caused to flow into the first channel 3. That is, the chemical solution or gas can be brought into contact with the fine particle T trapped in the adsorption portion S. Meanwhile, in FIG. 22, the upstream side of the first channel 3 is denoted by F1, and the downstream side thereof is denoted by F2.
The place in which the fourth channel 11 communicates with the first channel 3 and the shape of the fourth channel 11 are not particularly limited. Therefore, the fourth channel 11 can also be disposed at a position close to the fine vacuum hole 1, and each function of the upstream side (F 1 side) of the fourth channel 11 and the first channel 3 can be substituted. In addition, a plurality of fourth channels 11 or the fourth channels 11 plurally branched can also be disposed in communication with the first channel 3.

### <Second Embodiment of Base Body>

### [Base Body 30A]

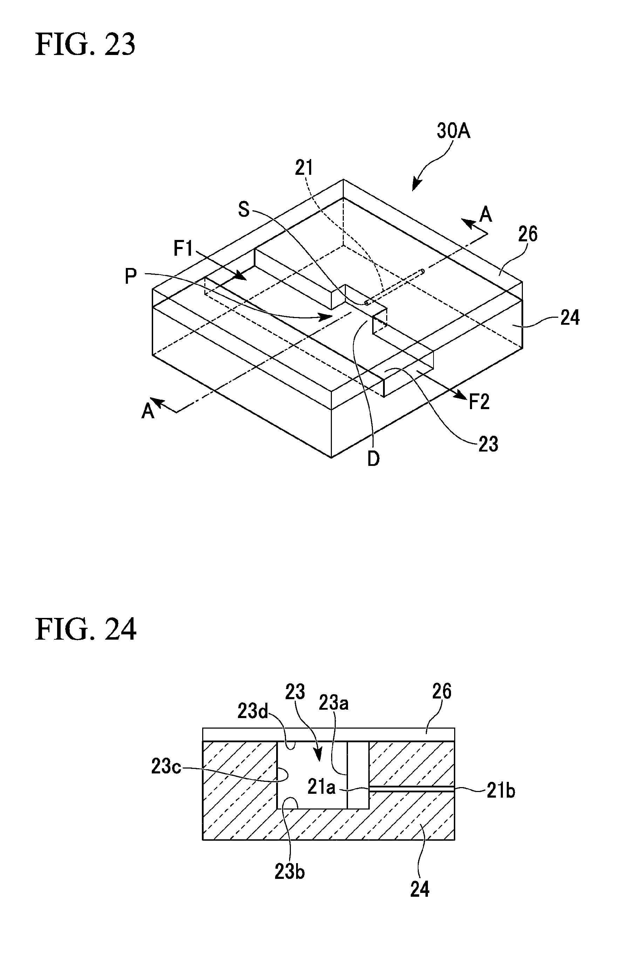

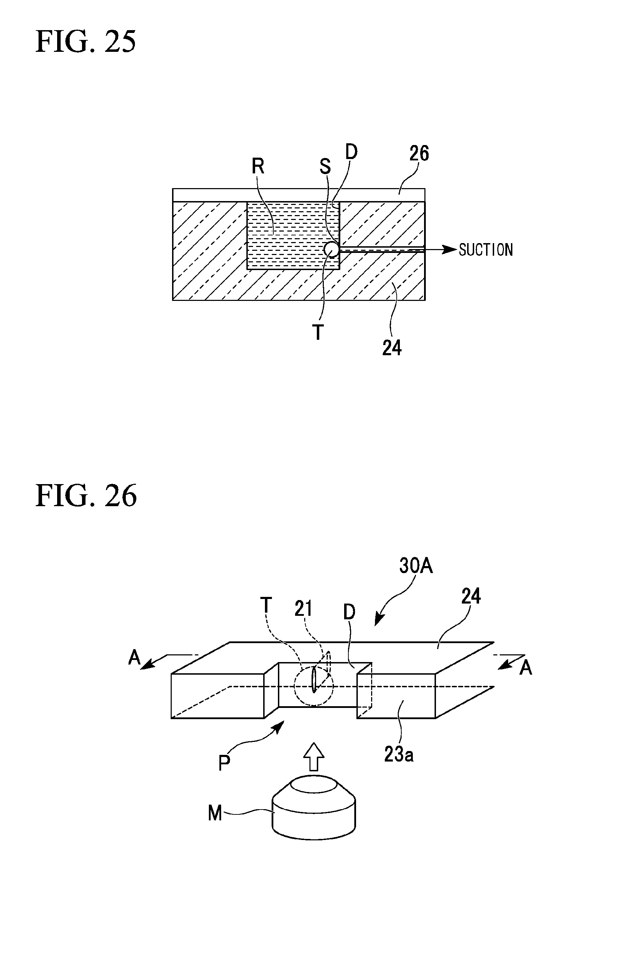

FIG. 23 is a perspective view illustrating a base body 30A which is a second embodiment of the base body according to the present invention. FIGS. 24 and 25 are schematic diagrams illustrating a cross-section taken along line A-A of FIG. 23.

The base body 30A is a base body including a fine vacuum hole 21 that traps a fine particle T. The base body 30A includes a channel 23, provided within a base member 24, which circulating a fluid R including the fine particle T, and the fine vacuum hole 21 that communicates between the channel 23 and the outside of the base member 24. In addition, at least a portion that configures the fine vacuum hole 21 in the base member 24 is formed of a single member. Furthermore, a substrate 26 that transmits visible light is bonded so as to cover the upper surface of the base member 24.
In addition, in the base body 30A, a slow flow portion P that slows the flow of the fluid R is provided at a position close to a first opening 21a of a fine vacuum hole 21 in an inner wall constituting the channel 23.

In the base body 30A, the channel 23 is provided on the upper surface side of the base member 24. In the channel 23, the fluid R including the fine particle T is circulated from the upstream side F1 to the downstream side F2.
A recess portion D constituting the slow flow portion P is provided at the lateral side 23 a of the channel 23, and an adsorption portion S having the first opening 21a of the fine vacuum hole 21 is formed at the lateral side 23a within the recess portion D. In addition, at least a portion of at least one of the upper surface 23d and the lower surface 23b of the channel 23 is constituted by the transparent member 26 (substrate 26) so as to optically observe the fine particle T trapped in the adsorption portion S.

FIG. 25 is a diagram illustrating a state where the fluid R is circulated through the channel 23 of the base body 30A, and the fine particle T contained in the fluid R is trapped in the adsorption portion S.

The fine particle T trapped by the base body of the present invention is contained in the fluid R, and is not particularly limited insofar as it can circulate through the channel 23. For example, the fine particle may include particles formed of a microorganism, a cell, or an organic substance, particles formed of an inorganic substance, and the like. The microorganism may include bacteria, fungi, molds, large-size viruses, and the like. The cell may include cells capable of suspension culture of erythrocytes, leukocytes, and the like. The particle formed of an organic substance may include particles constituted by macromolecules such as a resin or polysaccharide, active carbon particles, and the like. The particle formed of an inorganic substance may include metal particles such as a silica particle or a gold colloid particle.
The particle formed of an organic substance and the particle formed of an inorganic substance may be a functional particle in which antibody molecules or the like is bonded to the surface or inside thereof.
The shape of the particle formed of an organic substance and the shape of the particle formed of an inorganic substance are not particularly limited. For example, every particle having a three-dimensional shape such as a circle, a cube, a cuboid, a polyhedron, a doughnut-shaped solid, or a string-shaped solid is included in the fine particle of the present invention.
The size of the particle formed of an organic substance and the size of the particle formed of an inorganic substance may preferably be larger than the opening diameter (minor diameter) of a first end of the fine vacuum hole having the adsorption portion, and are not particularly limited. That is, the size may preferably be as large as the particles do not pass through the fine vacuum hole.

As the fine particle trapped by the base body of the present invention, a microorganism or a cell is preferable. The surface shape of the microorganism or the cell is relatively flexible. For this reason, the microorganism or the cell adheres sufficiently closely to the adsorption portion S, and thus can be adsorbed therein more strongly. Furthermore, when the fine particle is a microorganism or a cell, it is possible to perform a high-accuracy electrophysiological measurement.

As shown in FIG. 25, when the recess portion D constituting the slow flow portion P is provided at the lateral side 23 a of the channel 23, and the fine vacuum hole 21 opens on the lateral side 23 a within the recess portion D, the fine vacuum hole 21 and an object to be observed do not overlap each other at the time of an observation from the lower surface side of the base member 24 or the upper surface side of the base member 24, and thus the fine particle T can be easily observed.
FIG. 26 is a diagram illustrating a state where the fine particle T is optically observed while an objective lens M of a microscope moves closer to the lower surface of the base member 24. Meanwhile, in FIG. 26, only chief parts in the vicinity of the slow flow portion P in the base body 30A are shown.

When the trapped fine particle is observed from the lower surface of the base member, the distance between (working distance) an object to be observed and an objective lens is adjusted and the object to be observed is brought into focus. However, when the distance between the lower surface of the base member and the object to be observed is larger than the working distance required to be observed, the object to be observed cannot be brought into focus. In order to avoid such a situation, the thickness of the base member is reduced by polishing the lower surface of the base member, and thus it is possible to shorten the distance between the lower surface of the base member and the object to be observed. However, when the fine vacuum hole that opens on the lower surface 23b of the channel 23 is formed, the fine vacuum hole is present between the lower surface 23b of the channel 23 and the lower surface of the base member, and thus it may be difficult to reduce the thickness of the base member by polishing the lower surface of the base member. Therefore, as in the base body 30A, the fine vacuum hole preferably opens on the lateral side 23 a of the channel 23.

In the present invention, the fluid R including the fine particle T which is an object to be observed is not particularly limited, and includes, for example, blood, cell culture liquid, liquid for drink, river water, and the like. In addition, air including molds and the like is also included in the fluid R.

In the present invention, "circulation of the fluid R through the channel 23" means that the fluid R is put into the channel 23 from the outside of the base body 30A and is allowed to flow therethrough. The fluid R flowing into the channel may be continuously circulated through the channel 23, and the circulation thereof may be temporarily stopped, as necessary. The fluid R flowing through the channel 23 is finally discharged to the outside of the base body 30A, the same fluid R may be allowed to flow into the channel 23 again, as necessary, and may be circulated. This is applied to all the base bodies according to the present invention.

In the channel 23 of the base body 30A, a first inner wall surface 23 a (lateral side 23a) on which the slow flow portion P is provided and a second inner wall surface 23a (lateral side 23 a) on which the fine vacuum hole 21 opens are the same as each other. That is, the lateral side 23 a at which the recess portion D constituting the slow flow portion P is provided and the lateral side 23 a on which the first opening 21a of the fine vacuum hole 21 opens are formed on the same inner wall surface in the channel 23.

The slow flow portion P provided at a position close to the adsorption portion S has the recess portion D provided at the lateral side 23 a of the channel 23. The recess portion D is formed in a shape in which a portion of the channel 23 is surrounded, and the adsorption portion S is disposed at the lateral side 23a of the channel 23 in the surrounded portion.
Turbulent flows including components such as, for example, an upward flow, a downward flow, a backflow, and an eddy flow occur in the flow of the fluid R at a position close to the recess portion D in the slow flow portion P in which the recess portion D is disposed. That is, since the fluid R flows nonlinearly at the position close to the recess portion D, the flow of the fluid R becomes slow. The fine particle T in the fluid R is involved in the turbulent flow at the time of passing through the position close to the slow flow portion P. Therefore, in the channel of the present invention, the time for which the fine particle T stagnates at the position close to the slow flow portion P can be made longer than in a channel which is not provided with the slow flow portion P. As a result, the adsorption portion S provided at the position close to the slow flow portion P can easily trap the fine particle T.

In addition, after the fine particle T is trapped in the adsorption portion S, the fine particle T can be continued to be trapped more stably. The reason is as follows; since the adsorption portion S is provided in the innermost recess within the recess portion D, it is possible to surround the fine particle T within the recess portion D, and to protect the fine particle T by suppressing the influence of a main stream of the fluid R circulating through the channel 53.

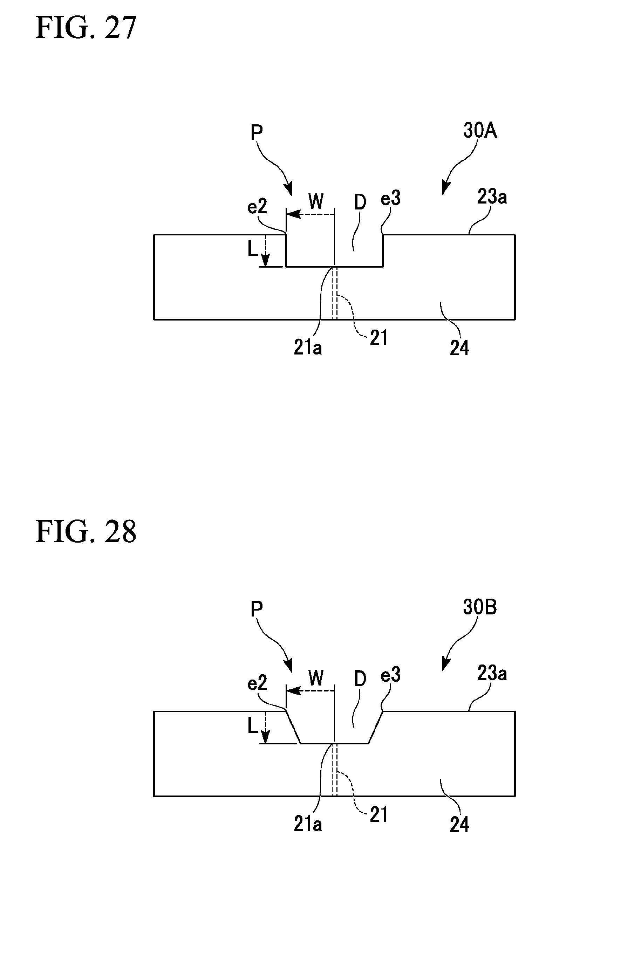

FIG. 27 is an enlarged view illustrating chief parts in the vicinity of the slow flow portion P when seen from the upper surface side of the base body 30A, and shows a longitudinal cross-section of the fine vacuum hole 21 along the extending direction of the channel 23. The longitudinal cross-section is a cross-section taken along line A-A of FIG. 26.
In the longitudinal cross-section of the fine vacuum hole 21 along the extending direction of the channel 23, the recess portion D has two corners. When the corner provided at a position close to the first opening 21a is set to a first corner e2, the corner provided at a position away from the opening is set to a second corner e3, and the distance between the first corner e2 and the first opening 21a is denoted by a depth L and a width W, the ratio of L/W is preferably in a range of 0.05 to 100. At this time, the depth L is preferably 1 µm to 100 µm, and the width W is preferably 0.1 µm to 50 µm.
Here, the depth L indicates the distance between the first corner e2 and the first opening 21a in a direction perpendicular to the extending direction of the channel 23, and the width W indicates the distance between the first corner e2 and the first opening 21a in the extending direction of the channel 23. In addition, the direction in which the fluid R flows does not matter.

In a case of the above-mentioned range, the adsorption portion S is provided within the sphere of the influence of the turbulent flow generated in the slow flow portion P, and thus the fine particle can be more easily trapped by the adsorption portion S. Further, when the trapped microorganism or cell T (fine particle) mitotically proliferates, daughter cells can be quickly broken away from the slow flow portion P by the turbulent flow. That is, the inside of the recess portion D can be prevented from being crowded by the daughter cells.

In the base body 30A shown in FIG. 23, the single member may not only include the fine vacuum hole 21, but also may constitute the entirety of the base member 24.
A material of the single member includes, for example, silicon, glass, silica, sapphire, or the like. Above all, the material is preferably an amorphous material which is not likely to be influenced by processing anisotropy due to a crystal orientation. When such a material is used, the processing accuracy of the fine vacuum hole 21 can be further increased, and the adsorption portion S can be formed with a diameter of the opening on the order of nanometers. For this reason, it is relatively easy to form a high-resistance seal between the trapped cell T and the adsorption portion S. At this time, when the adsorption portion S formed in the fine vacuum hole 21 is formed of glass, silica, or sapphire, this is preferable because a process of providing an insulation quality is not required. When a high-resistance seal is formed, and an electrophysiological measurement of the trapped cell T is performed, a method of measuring the potential difference (membrane potential) or the like between the inside and outside of the cell T can also be appropriately used in the base body to perform the measurement. When a material of which the adsorption portion S is formed is glass or silica, and the electrophysiological measurement of the trapped cell T is performed, a high-resistance seal of a gigaohm order (GΩ unit) between the cell T and the adsorption portion S is realized, and thus a higher-accuracy measurement can be made.

The material of the single member preferably transmits light having at least a portion of a wavelength in light having a wavelength of 0.1 1 µm to 10 µm.
Specifically, it is preferable to transmit at least a portion of normal light (wavelength of 0.1 1 µm to 10 µm) used as a processing laser. It is possible to form modified regions by irradiating, with such a laser, to the member as described below.
In addition, the material of the single member more preferably transmits light of a visible light region (wavelength of approximately 0.36 µm to approximately 0.83 µm). The light of a visible light region is transmitted, thereby allowing the trapped fine particle T to be easily observed through the single member using an optical method of an optical microscope or the like.
Furthermore, when the material of the single member transmits at least a portion of ultraviolet light or visible light (0.1 µm to 0.83 µm) in the light of the wavelength, it is possible to perform a fluorescent observation on the fine particle dyed or labeled by a fluorescent dye.
Meanwhile, "transmissive (transparent)" in the present invention refers to all the states where light is caused to be incident on the member, and transmitted light is obtained from the member.
In FIG. 23, the single member constituting the base member 24 is a transparent glass substrate.

As shown in FIGS. 24 and 25, the fine vacuum hole 21 communicates between the channel 23 and the outside of the base member 24. The first end 21a of the fine vacuum hole 21 is exposed (opens) onto the lateral side 23 a of the channel 23, and constitutes the adsorption portion S. The second end 21b of the fine vacuum hole 21 is exposed onto the lateral side of the base member 24.

In the present invention, when the fine vacuum hole 21 communicates between the channel 23 and the outside of the base member 24, the second opening 21b of the fine vacuum hole 21 may be exposed and opened onto the lateral side (outside) of the base member 24 as in the base body 30A of FIG. 23.

The fluid R within the channel 23 is suctioned from the second opening 21b side of the fine vacuum hole 21 which is exposed to the outside of the base member 24, thereby allowing the fine particle T contained in the fluid R to be trapped in the adsorption portion S. A dynamic force of the suction is not particularly limited, but the fluid can be suctioned by connecting a suction mechanism (not shown) such as, for example, a syringe or a pump to the second opening 21b of the fine vacuum hole 21.

In the base body 30A, the fine vacuum hole 21 is formed in the single glass substrate 24 (base member 24), and is a through hole which does not have a joint or a bonded surface. As a matter of course, a joint or a bonded surface is also not present in the adsorption portion S on the end of the fine vacuum hole 21. For this reason, it is possible to sufficiently increase adhesion between the fine particle T and the adsorption portion S. In addition, when an external force such as the deformation of the glass substrate 24 and erosion due to a chemical solution is applied, a bonded surface is not present in the fine vacuum hole 21 or in the vicinity of the fine vacuum hole 21, peeling or damage in the bonded surface does not occur. Thus, even when heating sterilization or chemical solution sterilization is repeatedly performed on the glass substrate 24, the glass substrate 24 is not damaged. This is a particularly excellent characteristic of the base body to routinely perform heating sterilization or chemical solution sterilization, and to trap a microorganism or a cell (fine particle T). Furthermore, since refractive index difference does not occur in the position close to the fine vacuum hole 21, light from the adsorption portion S is more likely to be condensed. Therefore, it is possible to easily observe the trapped fine particle. The term "adsorption portion S" as used herein refers to a region that comes into contact with or comes close to the fine particle T in the lateral side 23 a of the channel 23.

A hole, having the adsorption portion S, of the first opening 21a of the fine vacuum hole 21 in the lateral side 23 a of the channel 23 may have any of the shapes. For example, the hole can be circular, substantially circular, elliptic, substantially elliptic, rectangular, triangular, or the like. When the shape of the hole is circular or substantially circular, the diameter thereof may preferably be in a range of 0.02 µm to 5 µm. Above all, the diameter of a microorganism having a smaller size than that of a cell is more preferably in a range of 0.02 µm to 0.8 µm. When the shape is elliptic or substantially elliptic, and the minor diameter (shortest diameter) of the hole is in a range of 0.02 µm to 5 µm, the microorganism or the cell T can be trapped. That is, the minor diameter of the hole may preferably be set to such a size that the fine particle T cannot pass through the fine vacuum hole 1. For example, when an erythrocyte cell (6 to 8 µm) is trapped, the minor diameter may preferably be approximately 1 µm. When bacillus natto (bacillus subtilis; 0.7 µm to 2 µm) is trapped, the minor diameter may preferably be approximately 0.2 µm.

The range of the minor diameter is preferably 0.02 µm to 2 µm. Above all, in order to trap a microorganism having a smaller size than that of a cell, the diameter thereof is more preferably in a range of 0.02 µm to 0.8 µm.
When the diameter is less than a lower limit of the above-mentioned range, a suction force of the adsorption portion S is too weak, and thus there is a concern that the fine particle T is not able to be trapped. When the diameter exceeds an upper limit of the above-mentioned range, the fine particle T passes through the fine vacuum hole 21, and thus there is a concern that the fine particle is not able to be trapped.
On the other hand, the major diameter (longest diameter) of the hole may preferably be appropriately adjusted depending on the size of the fine particle T to be trapped, and includes, for example, a range of 0.2 µm to 10 µm. Furthermore, when an electrophysiological measurement is performed, the major diameter is preferably made smaller than the sizes of a cell and a microorganism, and includes, for example, a range of 0.2 µm to 5 µm.

Further, when the short side of the diameter of the hole is processed on the order of nanometers (nm unit) with a high degree of accuracy, it is possible to trap a microorganism having a smaller size than that of a cell. Therefore, in the related art, microorganisms observed as a group can be observed singly or only a few. Thereby, various biological characteristics of microorganisms which have not elucidated so far can be confirmed. Furthermore, by making the diameter of the adsorption portion of the hole very small, stable sealing between the cell membrane and the adsorption portion is realized at the time of measuring the electrophysiological characteristics of a cell, and thus the electrophysiological characteristics thereof can be stably measured.

In FIGS. 24 and 25, the fine vacuum hole 21 is formed so as to be substantially perpendicular to the lateral side 23a of the channel 23. However, the fine vacuum hole is not necessarily required to be substantially perpendicular thereto, but can be freely disposed in the single glass substrate 24 in accordance with the design of the base body 30A.
Further, the diameter at a position close to the first opening 21a of the fine vacuum hole 21 can also be made slightly larger than the diameter of the fine vacuum hole 21. At this time, since a portion of the fine particle T can be trapped in a state where it enters the first opening 21a of the fine vacuum hole 21, more stable trapping for a long period of time may be able to be performed even when a flow is present in the channel 23.
A plurality of fine vacuum holes 21 may be disposed in the base body 30A. Since each of the fine vacuum holes 21 is provided with the adsorption portion S, it is possible to trap a plurality of fine particles T.

In the base body 30A, the lower surface 23b of the channel 23 is provided in the base member 24 formed of a glass substrate. On the other hand, the upper surface of the channel 23 facing the lower surface 23b is covered with the substrate 26 formed of glass. The fine particle T trapped in the adsorption portion S can be observed from at least one of the lower surface 23b and the upper surface 23d using an optical observation mechanism such as a microscope. Meanwhile, the upper surface 23d is not necessarily required to be covered by a lid, but may have no lid in an opened state. Normally, since the fluid R is circulated within the channel 23 by applying a pressure, the upper surface 23d of the channel 23 is preferably covered with a lid.

A material constituting the upper surface 23d of the channel 23 is not particularly limited, and includes, for example, a resin substrate such as PDMS and PMMA, a silicon substrate, and a glass substrate. Particularly, when the material is a material that transmits visible light or ultraviolet light, this is preferable because the fine particle T can be easily observed. In addition, when the material of the substrate 26 is the same material as that of the base member 24, this is preferable because the base member 24 and the substrate 26 can be easily bonded to each other. In addition, the bonding may be performed using a known method.

When the periphery of the adsorption portion S in the channel 23 can be observed, it can be confirmed that the fine particle T is trapped in the adsorption portion S. In this case, for example, when the microorganism or the cell (fine particle) is trapped, a suction force of the fine vacuum hole 21 is made strong, and after it is trapped, the suction force is made weak to an extent that the cell membrane of the microorganism or the cell T is not damaged, thereby allowing the suction force to be properly adjusted depending on the state of the fine particle in the adsorption portion S.

When an electrophysiological measurement of the trapped microorganism or cell T is performed, for example, electrodes (not shown) may be disposed on the channel 23 and the fine vacuum hole 21b side, respectively. Alternatively, an external electrode through an extracellular buffer, an intracellular fluid or the like may be used. Since the adsorption portion S is formed of the single glass substrate 24, a high-resistance seal can be formed on the cell membrane of the cell T. Therefore, a patch-clamp method known in the related art can be applied. At this time, the hole diameter of the first opening 21a of the fine vacuum hole 21 having the adsorption portion S is made smaller than the hole diameter (approximately 2 µm to 4 µm) of a patch pipette or the like in the related art, thereby allowing an electrophysiological measurement having higher accuracy than in the related art to be performed.

### [Base Body 30B]

Another embodiment of the base body according to the present invention includes a base body 30B. The appearance of the base body 30B is substantially the same as that of the base body 30A, but is different therefrom in the shape of the recess portion D. The shape of the recess portion D of the base body 30B is shown in FIG. 28. In FIG. 28, the same components as those of the above-mentioned base body 30A are denoted by the same reference numerals and signs.
FIG. 28 is a longitudinal cross-section of the fine vacuum hole 21 along the extending direction of the channel 23 of the base body 30B.
That is, the drawing is a cross-section equivalent to FIG. 27 of the base body 30A. In this longitudinal cross-section, inclined planes inclined toward the lateral side 23 a of the channel 23 are formed between the lateral side 23 a of the channel 23 and the surface on which the first opening 21a is formed. The corner is formed between the lateral side 23a of the channel 23 and the inclined plane. In the base body 30B, the recess portion D is constituted by two corners, the inclined plane, and the surface on which the first opening 21a is formed.
In the recess portion D having the inclined planes, the distance between the two inclined planes facing each other in a direction perpendicular to the extending direction of the fine vacuum hole 21 gradually increases from the first opening 21a toward the channel 23. The recess portion D has the inclined planes, and thus a turbulent flow capable of being generated in the fluid R flowing along the vicinity of the slow flow portion P can be made different from a turbulent flow capable of being generated in the fluid R flowing along the vicinity of the slow flow portion P of the base body 30A.
Further, it is possible to regulate the type and strength of a turbulent flow generated in the fluid R flowing along the vicinity of the slow flow portion P by regulating the angle between the inclined plane in the recess portion D and the lateral side 23 a of the channel 23. The same effect as that of the base body 30A can also be obtained in the base body 30B.

In the longitudinal cross-section (FIG. 28) of the fine vacuum hole 21 along the extending direction of the channel 23 in the base body 30B, when the distance between the first opening 21a and the first corner e2 of the two corners e2 and e3 included in the recess portion D which is located on the side close to the first opening 21a of the fine vacuum hole 21 is denoted by depth L and width W, the ratio of L/W is preferably in a range of 0.05 to 100. At this time, the depth L is preferably 1 µm to 100 µm, and the width W is preferably 0.1 µm to 50 µm.
Here, the direction of the depth L is a direction perpendicular to the extending direction of the channel 23, and the direction of the width W is a direction parallel to the extending direction of the channel 23. In addition, the direction in which the fluid R flows does not matter.

### [Base Body 30C]

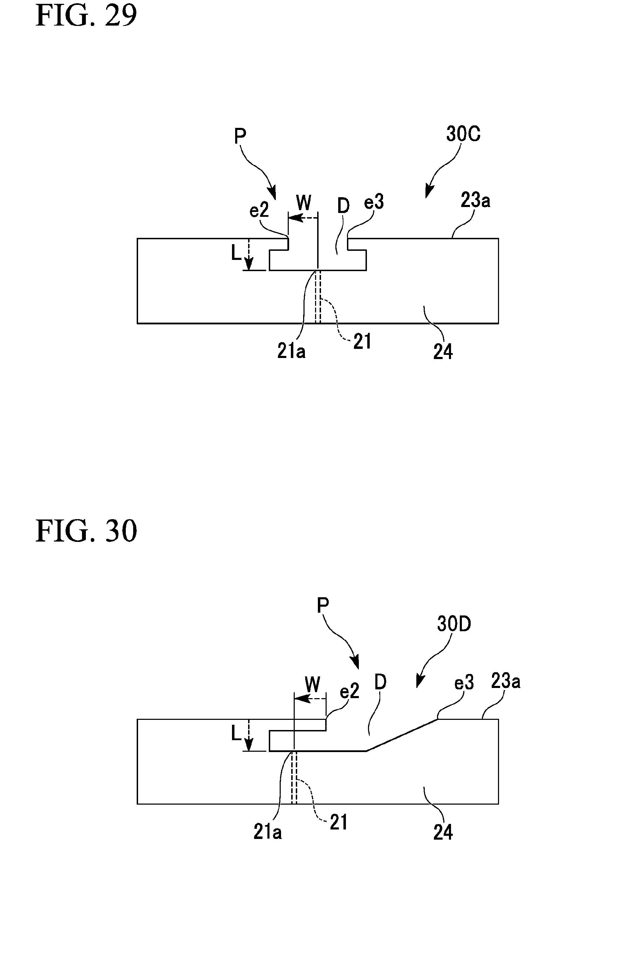

Another embodiment of the base body according to the present invention includes a base body 30C. The appearance of the base body 30C is substantially the same as that of the base body 30C, but is different therefrom in the shape of the recess portion D. The shape of the recess portion D of the base body 30C is shown in FIG. 29. In FIG. 29, the same components as those of the above-mentioned base body 30A are denoted by the same reference numerals and signs.
FIG. 29 is a longitudinal cross-section of the fine vacuum hole 21 along the extending direction of the channel 23 of the base body 30C.
That is, the drawing is a cross-section equivalent to FIG. 27 of the base body 30A. In this longitudinal cross-section, two corners e2 and e3 included in the recess portion D are provided with two protruding portions formed to be along the lateral side 23a. The corners e2 and e3 are located on the tip of each of the protruding portions and on the lateral side 23 a.
The protruding portions are formed in the recess portion D, and thus a turbulent flow capable of being generated in the fluid R flowing along the vicinity of the slow flow portion P can be made different from a turbulent flow capable of being generated in the fluid R flowing along the vicinity of the slow flow portion P of the base body 30A. More specifically, a tendency for the fluid R to stagnate within the recess portion D increases. The same effect as that of the base body 30A is also obtained in the base body 30C.

In the longitudinal cross-section (FIG. 29) of the fine vacuum hole 21 along the extending direction of the channel 23 in the base body 30C, when the distance between the first opening 21a and the first corner e2 of the two corners e2 and e3 included in the recess portion D which is located on the side close to the first opening 21a of the fine vacuum hole 21 is denoted by depth L and width W, the ratio of L/W is preferably in a range of 0.05 to 100. At this time, the depth L is preferably 1 µm to 100 µm, and the width W is preferably 0.1 µm to 50 µm.
Here, the direction of the depth L is a direction perpendicular to the extending direction of the channel 23, and the direction of the width W is a direction parallel to the extending direction of the channel 23.

### [Base Body 30D]

Another embodiment of the base body according to the present invention includes a base body 30D. The appearance of the base body 30D is substantially the same as that of the base body 30D, but is different therefrom in the shape of the recess portion D. The shape of the recess portion D of the base body 30D is shown in FIG. 30. In FIG. 30, the same components as those of the above-mentioned base body 30A are denoted by the same reference numerals and signs.
FIG. 30 is a longitudinal cross-section of the fine vacuum hole 21 along the extending direction of the channel 23 of the base body 30D.
That is, the drawing is a cross-section equivalent to FIG. 27 of the base body 30A. In this longitudinal cross-section, the recess portion D has two corners e2 and e3. One corner e2 is provided with a protruding portion formed along the inner wall portion 23a (lateral side 23a) of the channel 23. In addition, an inclined plane is formed at a position close to the other corner e3. The inclined plane is provided between the lateral side 23a of the channel 23 and the surface on which the first opening 21a is formed, and is inclined toward the lateral side 23 a of the channel 23.
The recess portion D has the above-mentioned shape, and thus a turbulent flow capable of being generated in the fluid R flowing along the vicinity of the slow flow portion P can be made different from a turbulent flow capable of being generated in the fluid R flowing along the vicinity of the slow flow portion P of the base body 30A. More specifically, a tendency for the fluid R to stagnate within the recess portion D increases, and thus it is possible to increase a tendency to protect the fine particle trapped in the first opening 21a of the fine vacuum hole 21 from a main stream of the fluid R flowing through the channel 23. The same effect as that of the base body 30A is also obtained in the base body 30D.

In the longitudinal cross-section (FIG. 30) of the fine vacuum hole 21 along the extending direction of the channel 23 in the base body 30D, when the distance between the first opening 21a and the first corner e2 of the two corners e2 and e3 included in the recess portion D which is located on the side close to the first opening 21a of the fine vacuum hole 21 is denoted by depth L and width W, the ratio of L/W is preferably in a range of 0.05 to 100. At this time, the depth L is preferably 1 µm to 100 µm, and the width W is preferably 0.1 µm to 50 µm. In FIG. 30, the first opening 21a of the fine vacuum hole 21 is located at a deeply recessed position of the recess portion D, but may be located at a front shallow position. In this case, an arrow denoting the width W may be directed opposite to an arrow shown in FIG. 30. In this case, the same is true of the above-mentioned suitable range of the ratio of L/W.
Here, the direction of the depth L is a direction perpendicular to the extending direction of the channel 23, and the direction of the width W is a direction parallel to the extending direction of the channel 23. In addition, the direction in which the fluid R flows does not matter.

### [Base Body 30E]

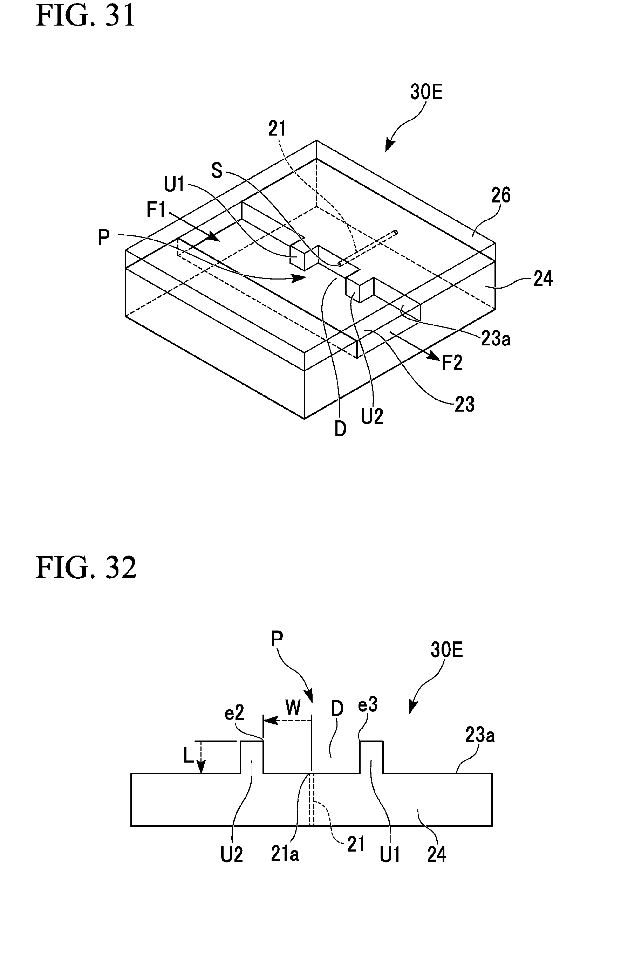

Another embodiment of the base body according to the present invention includes a base body 30E. FIG. 31 is a perspective view of the base body 30E. In FIG. 31, the same components as those of the above-mentioned base body 30A are denoted by the same reference numerals and signs.
A first protrusion U1 and a second protrusion U2 are disposed at the lateral side 23a of the channel 23 of the base body 30E, and the recess portion D is disposed between these two protrusions U1 and U2.
FIG. 32 shows a longitudinal cross-section of the fine vacuum hole 21 along the extending direction of the channel 23 of the base body 30E.
That is, the drawing is a cross-section equivalent to FIG. 27 of the base body 30A. In this longitudinal cross-section, the shape of the recess portion D is the same as the shape of the recess portion D of the base body 30A. However, since the two protrusions U1 and U2 are formed at positions close to the recess portion D, a turbulent flow capable of being generated in the fluid R flowing along the vicinity of the slow flow portion P can be made different from a turbulent flow capable of being generated in the fluid R flowing along the vicinity of the slow flow portion P of the base body 30A. More specifically, it is possible to further increase the extent of a turbulent flow capable of being generated in the fluid R flowing along the vicinity of the slow flow portion P. As a result, even when the fine particle flowing through the channel 23 is relatively large, the turbulent flow involves the fine particle, and thus it can be easy to pull and trap the fine particle in the adsorption portion S. The same effect as that of the base body 30A is also obtained in the base body 30E.
When the fluid R flows from the upstream side F1 to the downstream side F2, the same effect is obtained by providing only the first protrusion U1 located at the upstream side from the fine vacuum hole 21.

On the surfaces of which the first protrusion U1 and the second protrusion U2 face each other, the tip of the first protrusion U1 is provided with the corner e3, and the tip of the second protrusion U2 is provided with the corner e2.
In the longitudinal cross-section (FIG. 32) of the fine vacuum hole 21 along the extending direction of the channel 23 in the base body 30E, when the distance between the first opening 21a and the first corner e2 of the two corners e2 and e3 included in the recess portion D which is located on the side close to the first opening 21a of the fine vacuum hole 21 is denoted by depth L and width W, the ratio of L/W is preferably in a range of 0.05 to 100. At this time, the depth L is preferably 1 µm to 100 µm, and the width W is preferably 0.1 µm to 50 µm.
Here, the direction of the depth L is a direction perpendicular to the extending direction of the channel 23, and the direction of the width W is a direction parallel to the extending direction of the channel 23. In addition, the direction in which the fluid R flows does not matter.

### [Base Body 30F]

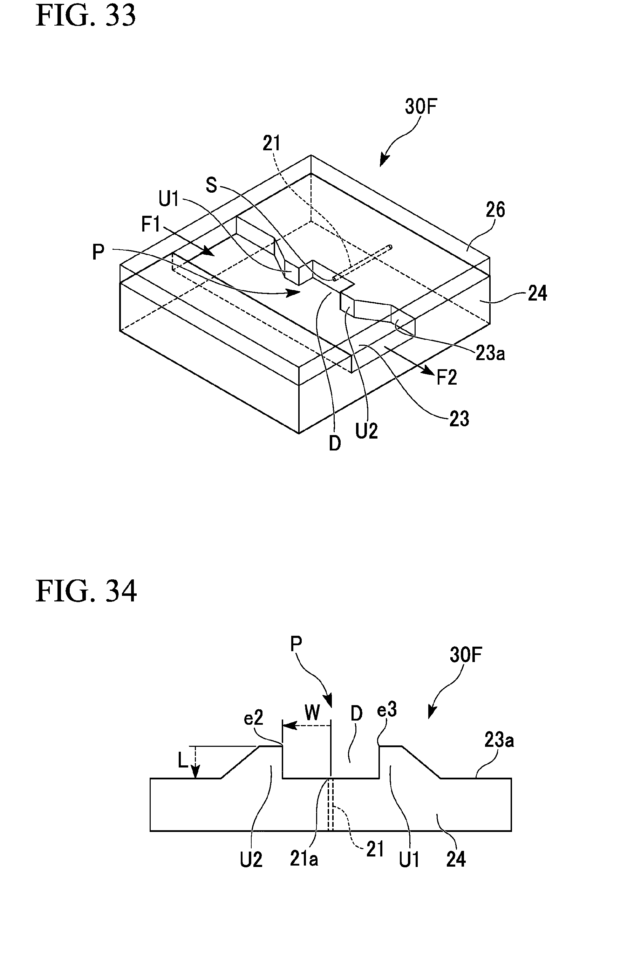

Another embodiment of the base body according to the present invention includes a base body 30F. FIG. 33 is a perspective view of the base body 30F. In FIG. 33, the same components as those of the above-mentioned base body 30A are denoted by the same reference numerals and signs.
The first protrusion U1 and the second protrusion U2 are disposed at the lateral side 23a of the channel 23 of the base body 30F, and the recess portion D is disposed between these two protrusions U1 and U2. The shapes of the two protrusions U1 and U2 are different from those of the protrusions U1 and U2 of the base body 30E mentioned above. The protrusions U1 and U2 of the base body 30F are formed in a trapezoidal shape in a longitudinal cross-section described later.

FIG. 34 shows a longitudinal cross-section of the fine vacuum hole 21 along the extending direction of the channel 23 of the base body 30F.
That is, the drawing is a cross-section equivalent to FIG. 27 of the base body 30A. In this longitudinal cross-section, the shape of the recess portion D is the same as the shape of the recess portion D of the base body 30A. However, since the two protrusions U1 and U2 are formed at positions close to the recess portion D, a turbulent flow capable of being generated in the fluid R flowing along the vicinity of the slow flow portion P can be made different from a turbulent flow capable of being generated in the fluid R flowing along the vicinity of the slow flow portion P of the base body 30A. More specifically, it is possible to further increase the extent of a turbulent flow capable of being generated in the fluid R flowing along the vicinity of the slow flow portion P. As a result, even when the fine particle flowing through the channel 23 is a relatively large fine particle, the turbulent flow involves the fine particle, and thus it can be easy to pull and trap the fine particle in the adsorption portion S. The same effect as that of the base body 30A is also obtained in the base body 30F.
When the fluid R flows from the upstream side F1 to the downstream side F2, the same effect is expected by providing only the first protrusion U1 located at the upstream side from the fine vacuum hole 21.

In the longitudinal cross-section (FIG. 32) of the fine vacuum hole 21 along the extending direction of the channel 23 in the base body 30F, when the distance between the first opening 21a and the first corner e2 of the two corners e2 and e3 included in the recess portion D which is located on the side close to the first opening 21a of the fine vacuum hole 21 is denoted by depth L and width W, the ratio of L/W is preferably in a range of 0.05 to 100. At this time, the depth L is preferably 1 µm to 100 µm, and the width W is preferably 0.1 µm to 50 µm.
Here, the direction of the depth L is a direction perpendicular to the extending direction of the channel 23, and the direction of the width W is a direction parallel to the extending direction of the channel 23. In addition, the direction in which the fluid R flows does not matter.

### [Base Body 30G]

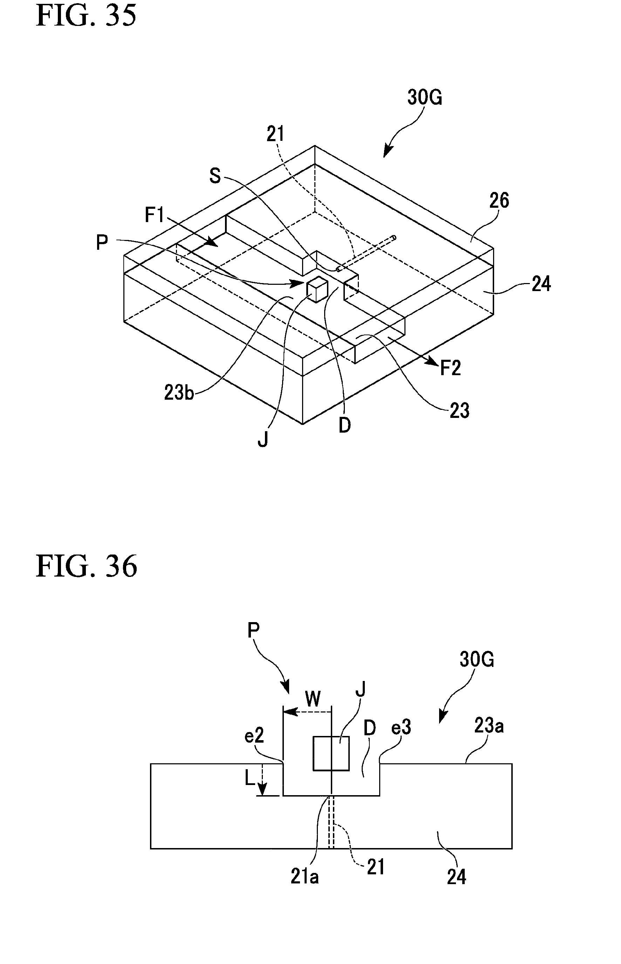

Another embodiment of the base body according to the present invention includes a base body 30G. FIG. 35 is a perspective view of the base body 30G. In FIG. 35, the same components as those of the above-mentioned base body 30A are denoted by the same reference numerals and signs.
In the lower surface 23b of the channel 23 of the base body 30G, a protruding portion J is disposed at a position facing the first opening 21a of the fine vacuum hole 21.

FIG. 36 is a longitudinal cross-section of the fine vacuum hole 21 along the extending direction of the channel 23 of the base body 30G.
That is, the drawing is a cross-section equivalent to FIG. 27 of the base body 30A. In this longitudinal cross-section, the shape of the recess portion D is the same as the shape of the recess portion D of the base body 30A. However, since the protruding portion J is formed at positions close to the recess portion D, a turbulent flow capable of being generated in the fluid R flowing along the vicinity of the slow flow portion P can be made different from a turbulent flow capable of being generated in the fluid R flowing along the vicinity of the slow flow portion P of the base body 30A. More specifically, it is possible to further increase the extent of a turbulent flow capable of being generated in the fluid R flowing along the vicinity of the slow flow portion P. As a result, even when the fine particle flowing through the channel 23 is relatively large, the turbulent flow involves the fine particle, and thus it can be easy to pull and trap the fine particle in the adsorption portion S. The same effect as that of the base body 30A is also obtained in the base body 30G.

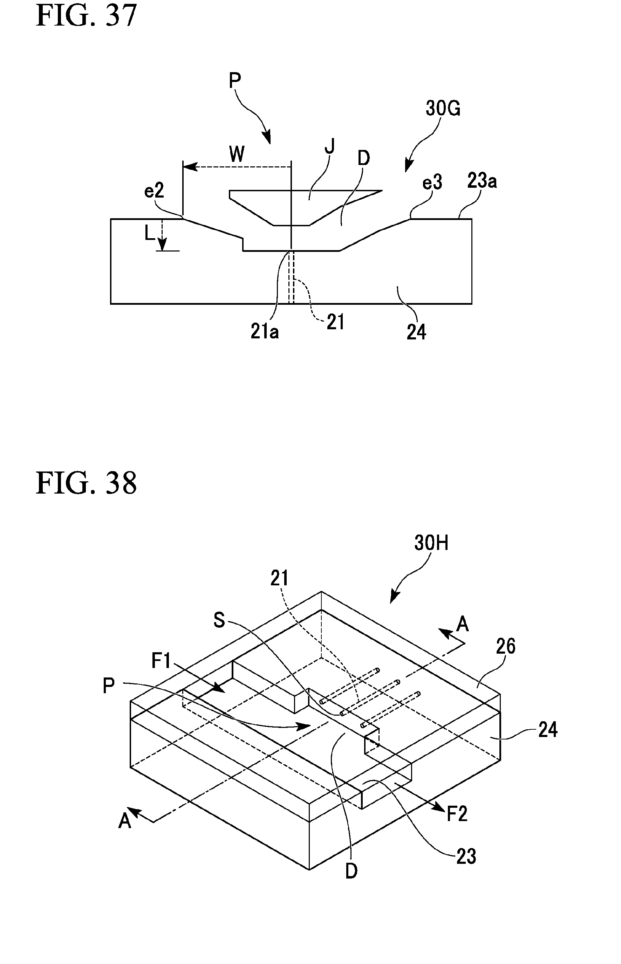

The shape of the protruding portion J is not particularly limited, but a shape such as a cube, a cuboid, a column, a triangle pole or the like can be adopted. In addition, the protruding portion J having a more complicate cross-sectional shape as shown in FIG. 37 may be disposed in the longitudinal cross-section.
The position at which the protruding portion J is disposed may be the inside of the recess portion D, and may be the outside of the recess portion D. When the protruding portion J is disposed at the inside of the recess portion D, it is preferable that the distance between the protruding portion J and the adsorption portion S is made sufficiently longer than the diameter of the trap fine particle. In addition, the number of protruding portions J to be disposed is not limited to one, and a plurality of protruding portions J may be disposed. In addition, in FIG. 35, although the protruding portion is formed on the lower surface of the channel, the surface on which the protruding portion J is disposed is not limited to the lower surface 23b of the channel 23, and may be preferably provided at a position close to the recess portion in an inner wall surface different from the inner wall surface in which the fine vacuum hole is formed.

### [Base body 30H]

Another embodiment of the base body according to the present invention includes a base body 30H. FIG. 38 is a perspective view of the base body 30H. In FIG. 38, the same components as those of the above-mentioned base body 30A are denoted by the same reference numerals and signs.
Three fine vacuum holes 21 are disposed parallel to each other at the lateral side 23a within the recess portion D of the base body 30H.

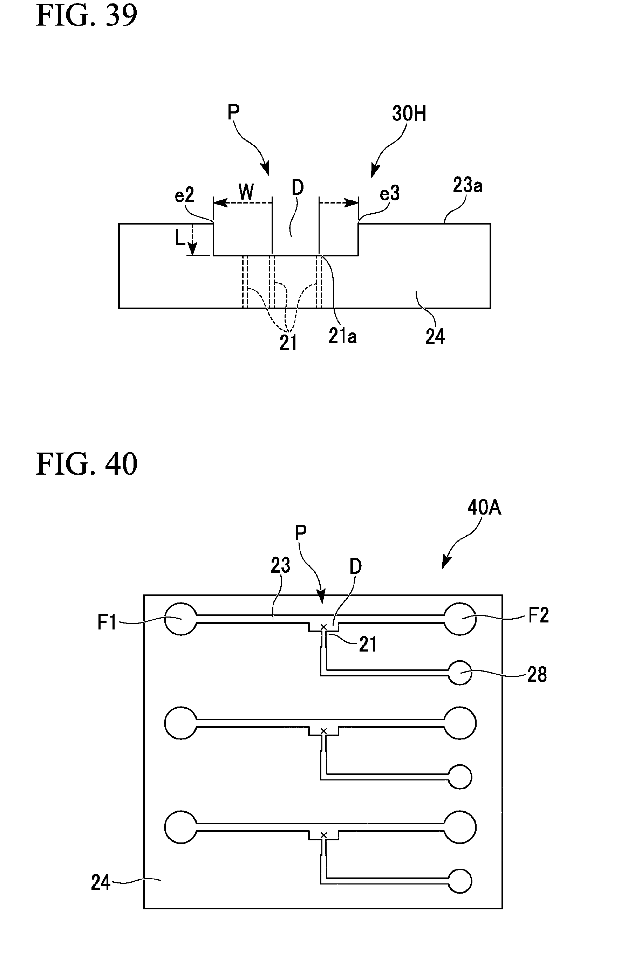

FIG. 39 shows a longitudinal cross-section of the fine vacuum hole 21 along the extending direction of the channel 23 of the base body 30H.
That is, the drawing is a cross-section equivalent to FIG. 27 of the base body 30A. In this longitudinal cross-section, since the first opening 21a each of the fine vacuum holes 21 is disposed at an interval longer than the diameter of the trap fine particle, the fine particles can be trapped in the adsorption portions S all at once. The same effect as that of the base body 30A is also obtained in the base body 30H.

In the longitudinal cross-section (FIG. 39) of the fine vacuum hole 21 along the extending direction of the channel 23 in the base body 30H, when the distance between the first opening 21a and the corner e2 (or corner e3) of the two corners e2 and e3 included in the recess portion D which is located on the side close to the first opening 21a of each of the fine vacuum holes 21 is denoted by depth L and width W, the ratio of L/W is preferably in a range of 0.05 to 100. At this time, the depth L is preferably 1 µm to 100 µm, and the width W is preferably 0.1 µm to 50 µm.
Here, the direction of the depth L is a direction perpendicular to the extending direction of the channel 23, and the direction of the width W is a direction parallel to the extending direction of the channel 23. In addition, the direction in which the fluid R flows does not matter.

In the base bodies 30A to 30H described above, an example in which only one channel 23 is disposed has been described, but as described in the above-mentioned first embodiment, the second to fifth channels, the electrode and the like can be disposed similarly in the base bodies 30A to 30H.

FIG. 40 is a schematic top view (perspective view seen from the upper surface) of a base body 40A which is an example of the base body according to the present invention. As a configuration of the slow flow portion P in the base body 40A, the configurations of the slow flow portion P in the above-mentioned base bodies 30A to 30H can be applied.
Three sets of composite channels including the first channel 23 and the third channel 28 are disposed in the glass substrate 24. Communication of the first channel 23 and the third channel 28 with each other through the fine vacuum hole 21 is as mentioned above. The position of the adsorption portion S is denoted by a sign of "X". The slow flow portion P having the recess portion D is provided at a position close to the adsorption portion S.
The fluid R flows in from the upstream side F1 of the first channel 23, and is circulated to the downstream side F2 of the third channel 28.

In the base body 40A, a plurality of third channels 28 can also be disposed in the single first channel 23. At this time, at least one or more fine vacuum holes 21 are provided in the single third channel 28. In this manner, when a plurality of third channels 28 are disposed in the single first channel 23, a suction mechanism (not shown) such as a syringe or a pump is connected independently to each of the third channels 28, thereby allowing the suction of the third channels 28 to be controlled independently. Therefore, the trapping of the fine particle T by the fine vacuum hole 21 can be controlled independently.

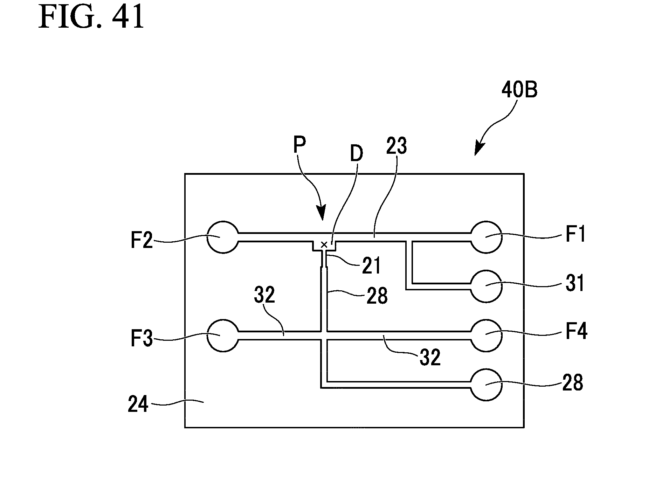

FIG. 41 is a schematic top view (perspective view seen from the upper surface) of a base body 40B which is an example of the base body according to the present invention. As a configuration of the slow flow portion P in the base body 40B, the configurations of the slow flow portion P in the above-mentioned base bodies 30A to 30H can be applied.

Communication of the first channel 23 and the third channel 28, disposed in the glass substrate 24, with each other through the fine vacuum hole 21 is as mentioned above. The position of the adsorption portion S is denoted by a sign of "X".
The fifth channel 32 disposed in the glass substrate 24 intersects the third channel 28 so as to communicate therewith. A desired chemical solution or gas is circulated from the upstream side F3 of the fifth channel 32 to the downstream side F4, thereby allowing the chemical solution or gas to be diffused and caused to flow into the third channel 28.
In addition, the chemical solution diffused into the third channel 28 can also be replaced with a gas.
The place in which the fifth channel 32 communicates with the third channel 28 and the shape of the fifth channel 32 are not particularly limited. Therefore, for example, the fifth channel 32 can also be disposed at a position close to the fine vacuum hole 21, and each function of the fifth channel 32 and the third channel 28 can be substituted. In addition, the fifth channel 32 can also be configured to have only the F3 side or F4 side, and any function of F3 or F4 can also be fulfilled by the third channel 28. A plurality of fifth channels 32 or the fifth channels 32 plurally branched may be disposed.

In addition, the fourth channel 31 disposed in the glass substrate 24 communicates with the first channel 23.
A desired chemical solution or gas is circulated from the fourth channel 31 to the first channel 23, thereby allowing the chemical solution or gas to be diffused and caused to flow into the first channel 23. That is, the chemical solution or gas can be brought into contact with the fine particle T trapped in the adsorption portion S. Meanwhile, in FIG. 41, the upstream side of the first channel 23 is denoted by F1, and the downstream side thereof is denoted by F2.
The place in which the fourth channel 31 communicates with the first channel 23 and the shape of the fourth channel 31 are not particularly limited. Therefore, the fourth channel 31 can also be disposed at a position close to the fine vacuum hole 21, and each function of the upstream side (F1 side) of the fourth channel 31 and the first channel 23 can be substituted. In addition, a plurality of fourth channels 31 or the fourth channels 31 plurally branched can also be disposed in communication with the first channel 23.

At stated above, descriptions have been made of the first embodiment (embodiment in which the first inner wall surface on which the slow flow portion is disposed and the second inner wall surface on which the opening of the fine vacuum hole is disposed are different from each other) of the base body according to the present invention, and the second embodiment (embodiment in which the first inner wall surface on which the slow flow portion is disposed and the second inner wall surface on which the opening of the fine vacuum hole is disposed are the same as each other). An embodiment in which the first embodiment and the second embodiment are appropriately combined can be implemented. In that case, the flow of the fluid R in the slow flow portion P disposed in the channel is made slower, and thus the fine particles can be trapped more easily.

Next, a method for manufacturing the base body according to the present invention will be described.

### <Method for Manufacturing Base Body (First Aspect)>

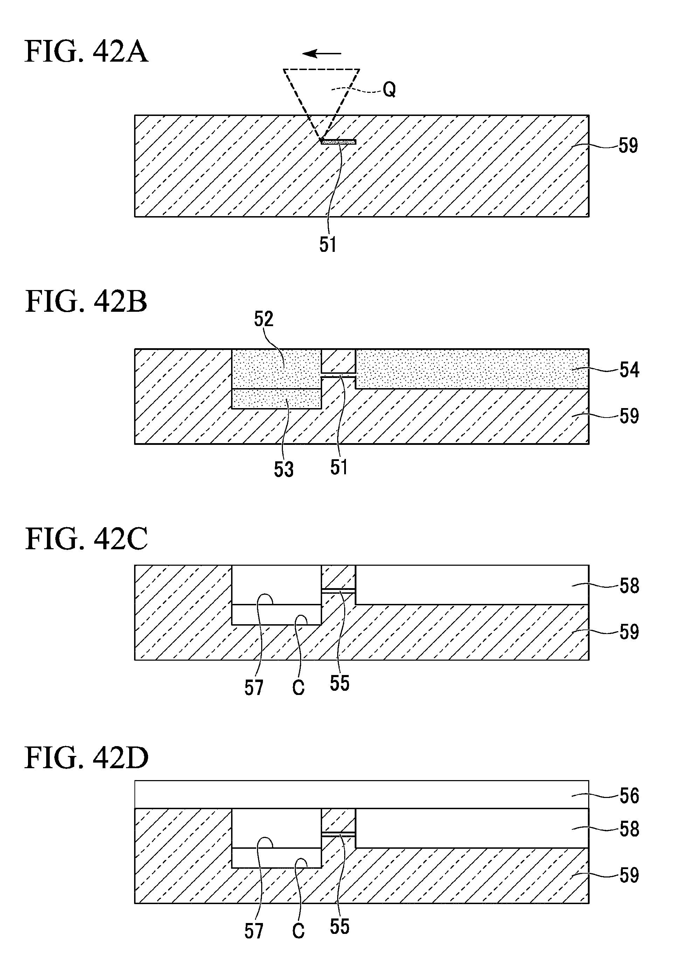

The above-mentioned base body 10G will be taken as an example to describe a first aspect of a manufacturing method according to the present invention. In this case, as shown in FIGS. 42A to 42D, the manufacturing method includes at least a process A1 (FIGS. 42A and 42B) of forming a first modified region 51, a second modified region 52, and a third modified region 53 by irradiating a region 51 in which a fine vacuum hole 55 is formed, a region 52 in which a first channel 57 is formed, and a region 53 in which a slow flow portion P is formed, in a single member 59, with a laser Q having a pulse duration of a picosecond order or less, and a process A2 (FIG. 42C) of removing the modified regions from the single member 59 by etching. Here, in the process of forming the first modified region 51, the second modified region 52, and the third modified region 53 using laser irradiation, the first modified region 51, the second modified region 52, and the third modified region 53 may be formed collectively by one-time of laser irradiation, or may be formed by multiple-times of laser irradiation.
In the process A1 in FIG. 42B, an example is also shown in which a modified region is formed in a region 54 in which a second channel 58 is formed. In addition, the slow flow portion P has a recess portion C formed in the inner wall surface of the first channel 57.

### [Process A1]

As the laser Q (laser light Q), it is preferable to use laser light having a pulse duration with a pulse duration of a picosecond order or less. For example, a titanium sapphire laser, a fiber laser having the pulse duration, and the like can be used. However, it is necessary to use a wavelength passing through the member 59. More specifically, it is preferably laser light of which transmittance to the member 59 is equal to or more than 60%.

Light of a general wavelength region (0.1 to 10 um) used as a processing laser can be applied to the laser Q (laser light Q). Above all, it is necessary to pass through the member 59 which is a member to be processed. A modified region can be formed in the member 59 by applying laser light of a wavelength passing through the member 59.

Materials of the member 59 include, for example, silicon, glass, silica, sapphire and the like. These materials are preferable because of their excellent processability at the time of forming the fine vacuum hole 55. Above all, the material is preferably an amorphous material which is not likely to be influenced by processing anisotropy due to a crystal orientation.
Further, when the fine particle or the like is optically observed using a microscope or the like, glass, silica, and sapphire are more preferable because they transmit visible light (wavelength of 0.36 µm to 0.83 µm).

In addition, the material of the member 59 preferably transmits light having at least a portion of a wavelength in light having a wavelength of 0.1 µm to 10 µm.
Specifically, it is preferable to transmit at least a portion of normal light (wavelength of 0.1 µm to 10 µm) used as a processing laser. It is possible to form modified regions by irradiating, with such a laser, to the member as described below.
In addition the material is more preferably transmits light of a visible light region (wavelength of approximately 0.36 µm to approximately 0.83 µm). The light of a visible light region is transmitted, thereby allowing the trapped fine particle T to be easily observed with the naked eye through the single member.
Meanwhile, "transmissive (transparent)" in the present invention refers to all the states where light is caused to be incident on the member, and transmitted light is obtained from the member.
In FIGS. 42A to 42D, the single member 59 is a transparent glass substrate (hereinafter, called the glass substrate 59).
Hereinafter, a case where the member 59 is a glass substrate will be described. However, even when the materials of the member 59 are other members, for example, silicon, silica, or sapphire, a process can be performed similarly. In the process A2 described later, silicon, silica, and glass having good processability are more suitable.

As the glass substrate 59, it is possible to use, for example, a glass substrate formed of silica, a glass substrate formed of silicate-based glass and borosilicate glass, and the like. A glass substrate formed of synthetic silica is suitable because of its good processability. In addition, the thickness of the glass substrate 59 is not particularly limited.

The method of performing irradiation with the laser light Q includes a method shown in FIG. 42A. That is, irradiation with the laser light Q is performed so as to condense and focus light on the inside of the glass substrate 59, and form modified regions by scanning the focus in an arrow direction as described below.
The modified region 51 having a desired shape can be formed in the region 51 to be the fine vacuum hole 55 by scanning the focus into in the inside of the glass substrate 59. Similarly, a modified region can be formed in the region 52 to be the first channel 57 and the region 53 to be the recess portion C so that the first channel 57 and the recess portion C have a desired shape by condensing and scanning the laser light Q.
The term "modified region" herein means a portion which has a low etching resistance and is removed selectively or preferentially by etching.

When the region 51 in which the fine vacuum hole 55 is formed is irradiated with the laser light Q, it is preferable that the irradiation intensity be set to a value close to a working threshold or less than a working threshold of the glass substrate 59, and that the polarization direction (electric field direction) of the laser light Q be perpendicular to the scanning direction.
In the present invention, the angle between the scanning direction of the laser light Q and the polarization direction of the laser light Q is preferably set to be more than 88° and equal to or less than 90°, more preferably set to be equal to or more than 88.5° and equal to or less than 90°, still more preferably set to be equal to or more than 89° and equal to or less than 90°, and particularly preferable set to be 90°.
This laser irradiation method is called a laser irradiation method α below.

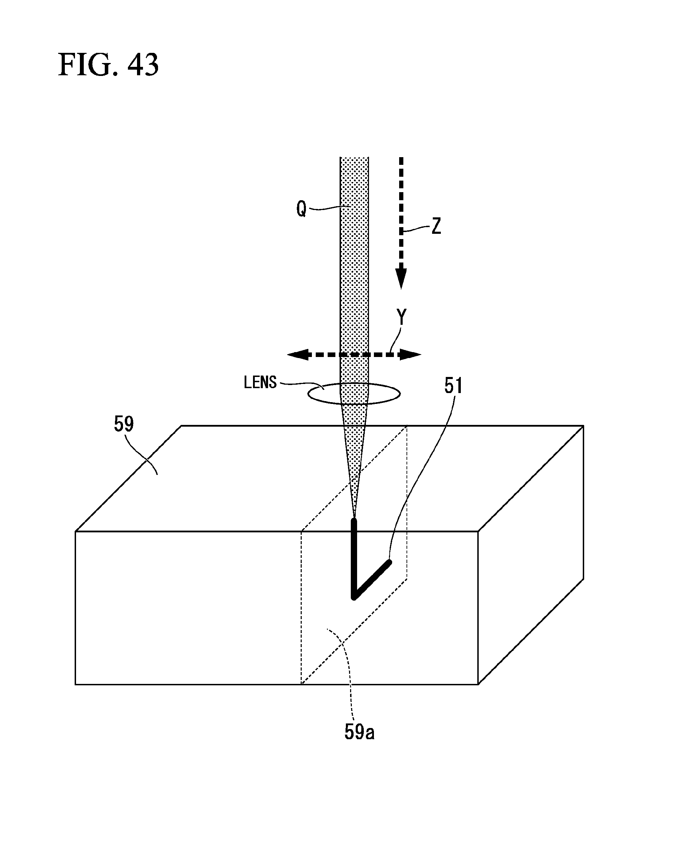

The laser irradiation method α will be described in FIG. 43. The propagation direction of the laser light Q is arrow Z, and the polarization direction (electric field direction) of the laser light Q is arrow Y. In the laser irradiation method α, a region to be irradiated with the laser light Q is set to be the inside of a plane surface 59a formed by the propagation direction of the laser light and the direction perpendicular to the polarization direction of the laser light. At the same time, laser irradiation intensity is set to a value close to a working threshold or less than a working threshold of the glass substrate 59.

The modified region 51 (modified region formed in the region 51 to be the fine vacuum hole 55) having a diameter of the opening on the order of nanometers within the glass substrate 59 can be formed by this laser irradiation method α. For example, the modified region 51, having a substantially elliptic cross-section, of which the minor diameter is approximately 20 nm and the major diameter is approximately 0.2 µm to 5 µm is obtained. In this substantially elliptic shape, the direction along the propagation direction of the laser becomes a major axis, and the direction along the electric field direction of the laser becomes a minor axis. The cross-surface may have a shape close to a rectangle according to the conditions of the laser irradiation.

When the laser irradiation intensity is set to equal to or more than the working threshold of the glass substrate 59, there is a possibility of the obtained modified region 51 being formed with a periodic structure. That is, a pulsed laser of a picosecond order or less is condensed to equal to or more than the working threshold and irradiation is performed, so that the interference between electron plasma waves and incident light occurs in a condensing portion, and thus a periodic structure which is perpendicular to laser polarization and has periodicity along the polarization direction is formed in a self-forming manner.

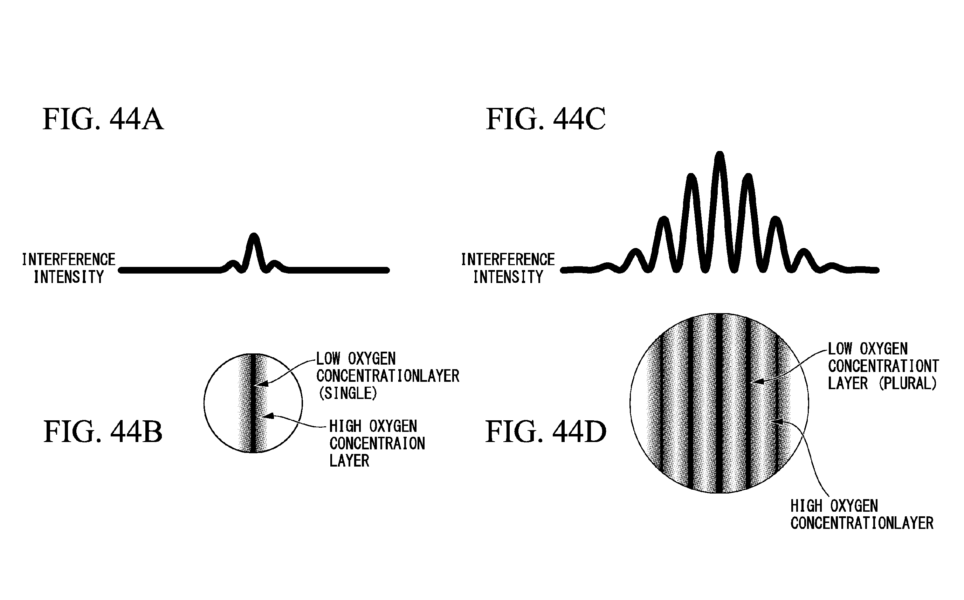

The formed periodic structure becomes a layer having a weak etching resistance. For example, a case of silica, a low oxygen concentration layer and a high oxygen concentration layer are periodically arranged (FIGS. 44C and 44D), and the etching resistance of a low oxygen concentration portion becomes weak, so that periodical recess portions and protrusions can be formed when etching is performed. Such periodical recess portions and protrusions are not required to form the fine vacuum hole 55 in the present invention.

On the other hand, as in the above-mentioned laser irradiation method α, when the laser irradiation intensity is set to be less than the working threshold of the glass substrate 59, and is set to be equal to or more than a lower limit of the laser irradiation intensity capable of lowering the etching resistance by modifying the glass substrate 59, the periodic structure is not formed, but one low oxygen concentration portion (layer having a low etching resistance) is formed by laser irradiation (FIGS. 44A and 44B). When this etching is performed, one fine vacuum hole 55 can be formed.

In the above-mentioned laser irradiation method α, the fine vacuum hole 55 can be formed in an elliptic or substantially elliptic shape. In addition, the minor diameter thereof can be controlled to the size on the order of nanometers by etching. The minor axis in an elliptic or substantially elliptic shape is made smaller than the fine particle size, thereby allowing the fine particle to be trapped. At this time, since the major axis thereof can be made larger than the size on the order of nanometers, a pressure loss of a fluid flowing in the fine vacuum hole 55 can be reduced. In addition, as a preliminary preparation for trapping the fine particle, it is necessary to fill the fluid in the fine vacuum hole 55 in advance. In this case, as the hole becomes finer, a capillary force increases. Therefore, an adverse effect that the fluid does not come out from the fine vacuum hole 55 to the outside such as a space may be generated. However, the fine vacuum hole 55 is formed in an elliptic or substantially elliptic shape, so that it is possible to suppress a capillary force even in the minor diameter enough to trap the fine particle, and to suppress an adverse effect that the fluid does not come out to the outside such as a space.

Even when one layer (low oxygen concentration portion in silica or glass) having a low etching resistance is formed by laser irradiation, the low oxygen concentration portion becomes a layer having extremely high etching selectivity. This fact has been found by the present inventors through keen examinations.
In the present invention, the layer having high etching selectivity is used as the modified region 51 which is a region to be the fine vacuum hole 55.

Therefore, the working threshold is defined as a lower limit (upper limit in a range of the laser irradiation intensity at which the periodic structure is not formed) of the laser irradiation intensity at which the periodic structure can be formed. Hereafter, in order to avoid confusion with a "working lower limit threshold value" described later, the working threshold is called a working upper limit threshold value.
In addition, the "lower limit (working lower limit threshold value) of the laser irradiation intensity capable of lowering the etching resistance by modifying the glass substrate 59" is a limit value capable of opening the fine vacuum hole 55 on the glass substrate 59 through an etching process. When the limit value is lower than the lower limit, the layer having a low etching resistance cannot be formed by laser irradiation, and thus the fine vacuum hole 55 is not opened.
That is, the "working upper limit threshold value" is a lower limit of the laser irradiation intensity at which interference between incident laser light and electron plasma waves generated due to interaction between the base member and the laser light occurs in the focus (condensed band) of the laser light with which the inside of the base member is irradiated, and a banded modified region can be formed in the base member in a self-forming manner by the interference.
In addition, the "working lower limit threshold value" is a lower limit of the laser irradiation intensity at which a modified region obtained by modifying the base member is formed on the focus (condensed band) of the laser light with which the inside of the base member is irradiated, and the etching resistance of the modified region can be lowered to an extent that etching can be performed selectively or preferentially by an etching process which is a post-process. It is not likely that a region irradiated with a laser at the laser irradiation intensity lower than the working lower limit threshold value is etched selectively or preferentially in the etching process which is a post-process. For this reason, in order to form a modified region serving a fine hole after etching, it is preferable that the working lower limit threshold value be set to the laser irradiation intensity of equal to or more than the lower limit threshold and equal to or less than the upper limit threshold.
The working upper limit threshold value and the working lower limit threshold value are generally determined by the wavelength of the laser light, the material (quality of the material) of the base member which is a laser irradiation object, and the laser irradiation conditions. However, when relative directions between the polarization direction and the scanning direction of laser light are different from each other, the working upper limit threshold value and the working lower limit threshold value are also different from each other to some extent. For example, when the scanning direction is perpendicular to the polarization direction, and the scanning direction is parallel to the polarization direction, the working upper limit threshold value and the working lower limit threshold value are different from each other. Therefore, in the wavelength of laser light to be used and the base member to be used, it is preferable to previously examine the working upper limit threshold value and the working lower limit threshold value when a relative relationship between the polarization direction and the scanning direction of the laser light is changed.

As the polarization, linear polarization has been described in detail. However, when a laser pulse having a few elliptic polarization components is used, it can be easily supposed that the same structure (modified region) is formed.

A method of scanning a focus of the laser light Q when a modified region is formed in the region 51 in which the fine vacuum hole 55 is formed is not particularly limited, but the modified region 51 capable of being formed by one-time continuous scanning is limited within the plane surface 59a formed by the one-dimensional direction perpendicular to the polarization direction (direction of arrow Y) and the propagation direction (direction of arrow Z) of the laser light Q. It is possible to form a modified region in an arbitrary shape within the direction of the plane surface.

Here, FIG. 43 shows a case where the propagation direction of the laser light Q is perpendicular to the upper surface of the glass substrate 59, but the propagation direction is not necessarily required to be perpendicular thereto. The upper surface may be irradiated with the laser Q at a desired incident angle.

Generally, the laser transmittance of the of the modified region is different from the laser transmittance of the non-modified region. Therefore, it is normally difficult to control a focal position of the laser light transmitted in the modified region. Therefore, it is preferable to form a modified region from a region located deep when seen from the surface irradiated with a laser.

In addition, by appropriately changing the polarization direction (direction of arrow Y) of the laser, the modified region 51 having an arbitrary shape in the three-dimensional direction can also be formed within the glass substrate 59.

In addition, as shown in FIG. 43, the modified region 51 may be formed by condensing the laser light Q using a lens and performing irradiation as mentioned above.
As the lens, for example, a refraction-type objective lens or a refraction-type lens can be used. Other than that, irradiation can also be performed by, for example, a Fresnel lens, a reflection-type lens, an oil immersion lens, and a water immersion lens. In addition, for example, when a cylindrical lens is used, the wide range of the glass substrate 59 can be irradiated with a laser all at once. In addition, for example, when a conical lens is used, the wide range of the glass substrate 59 in the vertical direction can be irradiated with the laser light Q all at once. However, when a cylindrical lens is used, the polarization of the laser light Q is required to be horizontal to a direction in which the lens has a curvature.

A specific example of laser irradiation conditions S includes the following various conditions. For example, a titanium sapphire laser (laser using a crystal obtained by doping sapphire with titanium as a laser medium) is used. In the irradiation laser light, for example, a wavelength of 800 nm and a repetition rate of 200 kHz are used, and the laser light Q is condensed and irradiation is performed at a laser scanning rate of 1 mm/second. The values of the wavelength, the repetition rate, and the scanning rate are an example. The present invention is not limited thereto, but the values can be arbitrarily changed.

As a lens used for condensation, it is preferable to use an objective lens of, for example, N.A. < less than 0.7. In order to form the finer vacuum hole 55, the pulse intensity when the glass substrate is irradiated is preferably power of a value close to the working upper limit threshold value, for example, approximately 80 nJ/pulse or less. In a case of power of the value or more, since periodic structures are formed and connected to each other by etching, it is difficult to form the fine vacuum hole 55 having a diameter of the opening on the order of nanometers. The vacuum hole having a micron-order diameter may be formed, or the periodic structure may be formed. In addition, even in a case of N.A. ≥ 0.7, processing can be performed, but a spot size is further reduced. Therefore, since laser fluence increases, laser irradiation at a smaller pulse amplitude is required.

On the other hand, when a region other than the region 51 in which the fine vacuum hole 55 is formed is irradiated with the laser light Q, the irradiation intensity may be set to a value sufficiently larger than the working upper limit threshold value of the glass substrate 59. The modified region (modified region 52) which is the region 52 to be the first channel 3 or the modified region (modified region 54) which is the region 54 to be the third channel 58 are normally processed in unit of micrometer (µm), and thus the focus of the laser light Q is not required to be narrowed in a size on the order of nanometers. For this reason, even when irradiation is performed at the irradiation intensity sufficiently larger than the working upper limit threshold value, the periodic structure is not formed.

### [Process A2]

Next, the modified region 51 formed in the process A1 is removed from the single glass substrate 59 by etching (FIG. 42C).
As an etching method, wet etching is preferable. The modified region formed in each of the regions 51, 52, 53, and 54 has a low etching resistance, and thus can be etched selectively or preferentially.

This etching method is a method using development in which the modified region is etched very rapidly as compared to the portion in which the glass substrate 59 is not modified. As a result, the fine vacuum hole 55, the first channel 57, the third channel 58, and the recess portion C can be formed according to the shape of the modified region.
An etching solution is not particularly limited, but it is possible to use, for example, a hydrofluoric acid (HF)-based solution, a fluoro-nitric acid-based mixed acid obtained by properly adding a nitric acid to a hydrofluoric acid, and the like. In addition, it is also possible to use other chemical solutions depending on the material of the member 59.

As a result of the etching, the fine vacuum hole 55 having a diameter of the opening on the order of nanometers can be formed at a predetermined position within the glass substrate 59 so as to communicate between the first channel 57 and the third channel 58. In addition, the recess portion C can be formed at a predetermined position of the first channel 57.

It is possible to form a through hole, having a substantially elliptic cross-section, of which the minor diameter is approximately 20 nm to 200 nm and the major diameter is approximately 0.2 µm to 5 µm, for example, as the size of the fine vacuum hole 55. The cross-surface may have a shape close to a rectangle according to the conditions of the etching process.

It is possible to decrease or increase the size difference between the modified region 51 and the fine vacuum hole 55 by adjusting the processing time of the wet etching.
Logically, it is also possible to set the minor diameter to several nm to tens of nm by shortening the processing time. On the contrary, it is also possible to set the minor diameter to approximately 1 µm to 2 µm and to set the major diameter to approximately 5 µm to 10 µm by lengthening the processing time.

Next, the upper surface of each channel is formed by bonding the member 56 to the upper surface of the glass substrate 59 so as to cover the first channel 57 and the third channel 58 which are formed (FIG. 42D).

A method of bonding the member 56 to the upper surface of the glass substrate 59 may be performed using a known method depending on the material of the member 56.
In addition, at the time of the bonding, an electrode, a wiring or the like for an electrophysiological measurement can also be appropriately installed within the first channel 57 and the third channel 58.

A material of the member 56 is not particularly limited, but a resin substrate such as PDMS and PMMA, or a glass substrate can be used. In addition, the member 56 may be formed of a material that transmits light (for example, ray of visible light) used for observation, and may be formed of a material that does not transmit light. When only the trapping of the fine particle in intended, it is not necessarily required to transmit light used for observation. A member that transmits light used for observation may be preferable because the observation can be performed optically from the upper surface.

As the etching in the process A2, wet etching or dry etching can be applied. In the wet etching, it is most preferable to use, for example, a hydrofluoric acid of equal to or less than 1%, but other acids or basic substances may be used.

Isotropic etching method in the dry etching include, for example, various types of dry etching methods such as barrel-type plasma etching, parallel plate-type plasma etching, or down-flow-type chemical dry etching.

In anisotropic dry etching methods, for example, parallel plate-type RIE, magnetron-type RIE, ICP-type RIE, NQD-type RIE or the like can be used as methods using reactive ion etching (hereinafter, RIE), and etching using, for example, a neutral beam can be used in addition to RIE When the anisotropic dry etching method is used, an ion mean free path is shortened by a method of raising process pressure, and thus processing close to isotropic etching can also be performed.

As gases to be used, for example, gases capable of chemically etching materials such as a fluorocarbon-based gas, an SF-based gas, a CHF₃ gas, a fluorine gas, or a chlorine gas can be mainly used by appropriately mixing gases such as other oxygen, argon, and helium. Processing using other dry etching methods can also be performed.

### <Method for Manufacturing Base Body (Second Aspect)>

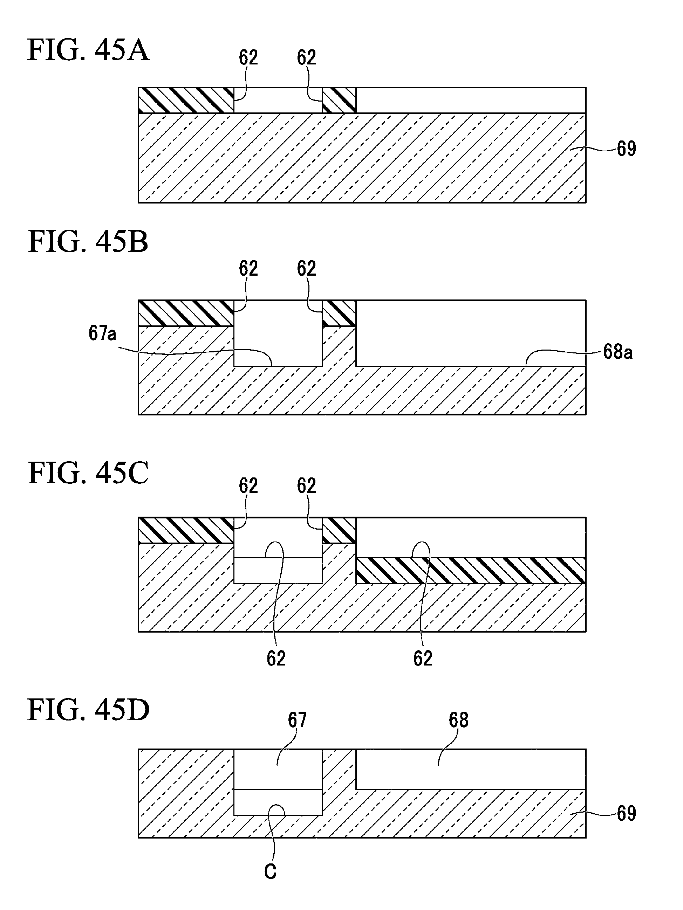

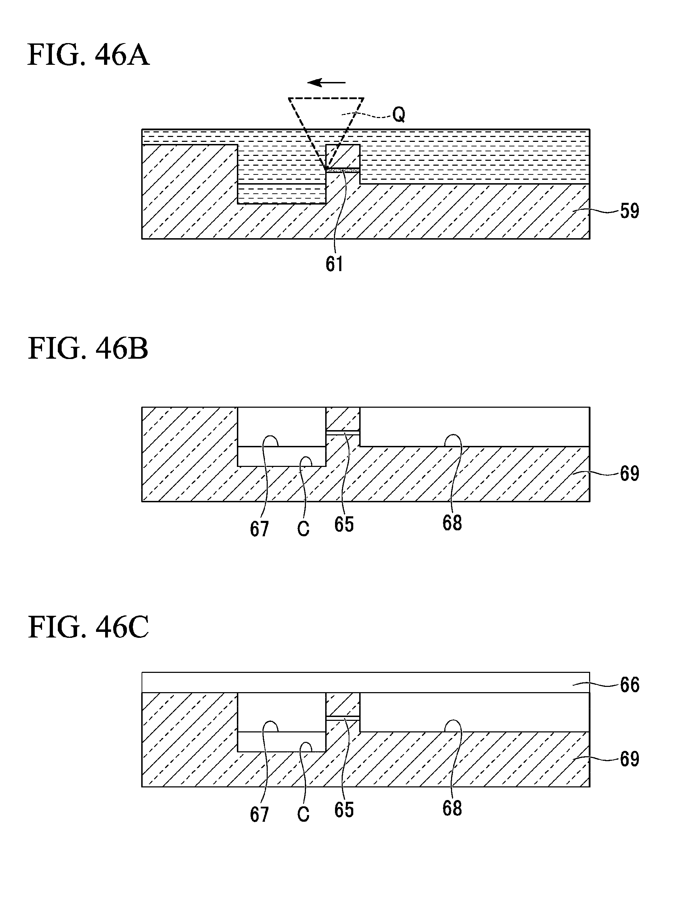

The above-mentioned base body 10G will be taken as an example to describe a second aspect of a manufacturing method according to the present invention. In this case, as shown in FIGS. 45A to 45D, the manufacturing method includes at least a process B1 of forming a first channel 67 and a slow flow portion P in a single member 69 ( FIG.45A to D), a process B2 of forming a modified region 61 in a region 61 in which a fine vacuum hole 65 of the single member 69 is formed, by irradiating the region with a laser having a pulse duration of a picosecond order or less (FIG. 46A), and a process B3 of removing the modified region 61 from the single member 69 by etching (FIG. 46B).
Meanwhile, it does not matter which of the process B1 and the process B2 is first performed.

Materials of the member 69 include, for example, silicon, glass, silica, sapphire and the like. These materials are preferable because of their excellent processability at the time of forming the fine vacuum hole 65. Above all, the material is preferably an amorphous material which is not likely to be influenced by processing anisotropy due to a crystal orientation.
Further, when the fine particle or the like is optically observed using a microscope or the like, glass, silica, and sapphire are more preferable because they transmit visible light (wavelength of 0.36 µm to 0.83 µm).

In addition, the material of the member 69 preferably transmits light having at least a portion of a wavelength in light having a wavelength of 0.1 µm to 10 µm.
Specifically, it is preferable to transmit at least a portion of normal light (wavelength of 0.1 µm to 10 µm) used as a processing laser. It is possible to form modified regions by irradiating, with such a laser, to the member as described below.
In addition the material is more preferably transmits light of a visible light region (wavelength of approximately 0.36 µm to approximately 0.83 µm). The light of a visible light region is transmitted, thereby allowing the trapped fine particle T to be easily observed with the naked eye through the single member.
Meanwhile, "transmissive (transparent)" in the present invention refers to all the states where light is caused to be incident on the member, and transmitted light is obtained from the member.
In FIGS. 45A to 45D, the single member 69 is a transparent glass substrate (hereinafter, called the glass substrate 69).

### [Process B1]

In the process B1, a resist 62 is patterned and disposed on the upper surface of the glass substrate 69 by photolithography (FIG. 45A). Subsequently, a region in which the resist 62 on the upper surface of the glass substrate 69 is not disposed is etched and removed by dry etching, wet etching, or a method such as sand blast until reaching a predetermined depth (FIG. 45B). Finally, when the resist 62 is peeled off, the glass substrate 69 in which the first channel 67 and the third channel 68 are formed is obtained.

Next, the resist 62 is formed by photolithography again in a place other than the region in which the recess portion C is formed (FIG. 45C). Subsequently, a region in which the resist 62 on the upper surface of the glass substrate 69 is not formed is eroded and removed by dry etching, wet etching, or a method such a sand blast until reaching a predetermined depth. Finally, when the resist 62 is peeled off, the first channel 67, the third channel 68, and the glass substrate 69 in which the recess portion C is formed are obtained (FIG. 45D).

In the process B1, the process of forming the first channel 67 and the third channel 68 and the process of forming the recess portion C can also be replaced with each other. That is, first, a resist is formed so that a region in which the recess portion C is formed is opened on the glass substrate, and the glass substrate of the opened region is etched up to a depth of the recess portion C. Thereafter, the recess portion C is formed in the glass substrate by removing the resist. Next, a resist is formed so that regions to be the first channel 67 and the third channel 68 are opened, and the glass substrate of the opened regions is etched, to thereby form the first channel 67 and the third channel 68.

In the process B1, in order to form the first channel 67 and the third channel 68, a drilling mechanism such as a drill (laser drill) may be used.

### [Process B2]

Next, a region in which the fine vacuum hole 65 of the single glass substrate 69 is formed is irradiated with the laser Q having a pulse duration of a picosecond order or less, to thereby form the modified region 61 in the region.
Specifically, the above process can be performed similarly to the process A1 in the first aspect of the manufacturing method according to the present invention. At this time, since the laser light Q is condensed on a location exposed to the lateral side of the first channel 67 and the third channel 68 and the location is irradiated therewith to form the modified region 61, it is more preferable to irradiate the location with the laser light Q by liquid immersion exposure (FIG. 46A). It is possible to increase the accuracy of the shape (shape of the end of the fine vacuum hole 65) of the modified region 61 which is formed at a location exposed to the lateral side.

### [Process B3]

Subsequently, the modified region 61 formed in the process B2 is removed from the single glass substrate 69 by etching (FIG. 46B).
As an etching method, wet etching is preferable. The modified region 61 having a cross-section exposed to the lateral side of the first channel 67 and the third channel 68 has a low etching resistance, and thus can be etched selectively or preferentially.
Specifically, the above process can be performed similarly to the process A2 in the first aspect of the manufacturing method according to the present invention.

As a result of the etching, the fine vacuum hole 65 having a diameter of the opening on the order of nanometers can be formed at a predetermined position within the glass substrate 69 so as to communicate between the first channel 67 and the third channel 68.

Next, the upper surface of the first channel 67 and the upper surface of the third channel 68 can be formed by bonding the member 66 to the upper surface of the glass substrate 69 so as to cover the first channel 67 and the third channel 68 which are formed (FIG. 46C).

A method of bonding the member 66 to the upper surface of the glass substrate 69 may be performed using a known method depending on the material of the member 66.
In addition, at the time of the bonding, an electrode, a wiring or the like for an electrophysiological measurement can also be appropriately installed within the first channel 67 and the third channel 68.

A material of the member 66 is not particularly limited, but a resin substrate such as PDMS and PMMA, or a glass substrate can be used. In addition, the member 66 may be formed of a material that transmits light (for example, ray of visible light) used for observation, and may be formed of a material that does not transmit light. When only the trapping of the fine particle in intended, it is not necessarily required to transmit light used for observation. A member that transmits light used for observation may be preferable because the observation can be performed optically from the upper surface.

### <Method for Manufacturing Base Body (Third Aspect)>

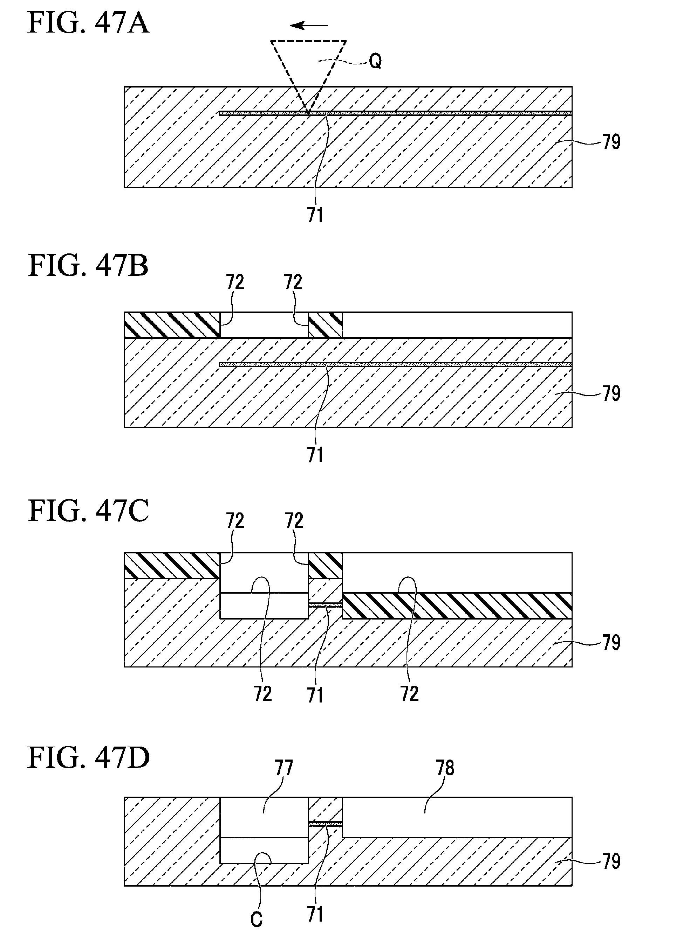

The above-mentioned base body 10A will be taken as an example to describe a third aspect of a manufacturing method according to the present invention. In this case, as shown in FIGS. 47A to 47D, the manufacturing method includes at least a process C1 of irradiating, at least, a region 71 in which a fine vacuum hole of a single member 79 is formed and a portion of a region in which a first channel 77 is formed, with a laser having a pulse duration of a picosecond order or less, to form a modified region 71 in each of the regions (FIG. 47A), a process C2 of forming the first channel 77 and the slow flow portion P in the single member 79 (FIG. 47B to D), and a process C3 of removing the modified region 71 from the single member 79 by etching.

A material of the member 79 may be the same as that of the member 59 described in the above-mentioned first aspect of the manufacturing method according to the present invention. In FIGS. 47A to 47D, the single member 79 is a transparent glass substrate (hereinafter, called the glass substrate 79).

### [Process C1]

The region 71 in which the fine vacuum hole of the single glass substrate 79 is formed, the region in which the first channel 77 is formed, and the region in which the third channel 78 is formed are irradiated with the laser Q having a pulse duration of a picosecond order or less, to form the modified region 71 in these region. Specifically, the same process as that of the process A1 in the first aspect of the manufacturing method according to the present invention may be performed.

### [Process C2]

Next, a resist 72 is patterned and formed on the upper surface of the glass substrate 79 by photolithography (FIG. 47B). Subsequently, a region in which the resist 72 on the upper surface on the glass substrate 79 is not formed is eroded and removed by dry etching, wet etching, or a method such as sand blast until reaching a predetermined depth. At this time, a portion of the modified region 71 is removed by etching. Here, when the resist 72 is peeled off, the first channel 77, the third channel 78, and the glass substrate 79 in which the modified region 71 is formed are obtained.

According to this method, a first end and a second end of the modified region 71 can be exposed to the lateral side of the first channel 77 and the lateral side of the third channel 78 which are formed by the etching.
By wet etching the modified region 71 as described above, it is possible to form a fine vacuum hole having an opening at the lateral side of the first channel 77 and the lateral side of the third channel 78. In addition, the fine vacuum hole communicates between the first channel 77 and the third channel 78.

Therefore, as compared to the method of forming the modified region only in the region to be the fine vacuum hole by laser irradiation, the laser irradiation position in the process C1 can be easily controlled in the method of the third aspect. That is, the position of the opening of the fine vacuum hole can be adjusted by the resist pattern and etching without being required to be adjusted by the laser irradiation.

In the process C2, as a method of eroding and removing the region in which the resist 72 is not disposed until reaching a predetermined depth, dry etching is preferable.
When the dry etching is used, it is possible to form the first channel 77, and to form the recess portion C constituting the slow flow portion P in the first channel 77. This is because the recess portion C is formed preferentially when the first channel 77 is formed, as a result that the modified region 71, formed in the process C1, which is formed in the region in which the first channel 77 is formed is removed preferentially by the dry etching. The diameter of the modified region 71 becomes smaller as the fine vacuum hole can be formed. However, in the dry etching method, since the above-mentioned etching gas enlarges the modified region 71 to advance the etching, the recess portion C having a sufficient capacity can be formed. The conditions of the dry etching at this time can be performed under normal conditions.
In this manner, when the first channel 77 and the recess portion C are formed by the dry etching, the glass substrate 79 having a cross-section shown in FIG. 47D is obtained.
Meanwhile, a description has been made of the formation of the recess portion C constituting the slow flow portion P in the first channel 77. However, even when the modified region 71 is formed in the region in which the third channel 78 is formed, a recess portion constituting the slow flow portion is formed in the third channel 78 similarly.

Next, when the recess portion C formed by the dry etching method does not have a desired size, the resist 72 is formed by photolithography again in a place other than the region in which the recess portion C is formed (FIG. 47C). Subsequently, a region in which the resist 72 on the upper surface of the glass substrate 79 is not disposed is eroded and removed by dry etching, wet etching, or a method such as sand blast until reaching a predetermined depth. When the resist 72 is peeled off, the first channel 77, the third channel 78, the recess portion C constituting the slow flow portion P, and the glass substrate 79 in which the modified region 71 formed in the region to be the fine vacuum hole 75 is disposed are obtained (FIG. 47D).

In the process C2, in order to form the first channel 77 and the third channel 78, a drilling mechanism such as a drill (laser drill) may be used.

### [Process C3]

Subsequently, the modified region 71 formed in the process C1 and the process C2 is removed from the single glass substrate 79 by etching (not shown).
As an etching method, wet etching is preferable. The modified region 71 having a cross-section exposed to the lateral side of the first channel 77 and the third channel 78 has a low etching resistance, and thus can be etched selectively or preferentially.
Specifically, the above process can be performed similarly to the process A2 in the first aspect of the manufacturing method according to the present invention.

As a result of the etching, the fine vacuum hole having a diameter of the opening on the order of nanometers can be formed at a predetermined position within the glass substrate 79 so as to communicate between the first channel 77 and the third channel 78.

Next, the upper surface of the first channel 77 and the upper surface of the third channel 78 can be formed by bonding the member to the upper surface of the glass substrate 79 so as to cover the first channel 77 and the third channel 78 which are formed by the above process (not shown).

A method of bonding the member to the upper surface of the glass substrate 79 may be performed using a known method depending on the material of the member 76.
In addition, at the time of the bonding, an electrode, a wiring or the like for an electrophysiological measurement can also be appropriately installed within the first channel 77 and the third channel 78.

A material of the member is not particularly limited, but a resin substrate such as PDMS and PMMA, or a glass substrate can be used. In addition, the member may be formed of a material that transmits light (for example, ray of visible light) used for observation, and may be formed of a material that does not transmit light. When only the trapping of the fine particle in intended, it is not necessarily required to transmit light used for observation. A member that transmits light used for observation may be preferable because the observation can be performed optically from the upper surface.

### <Method for Manufacturing Base Body (Fourth Aspect)>

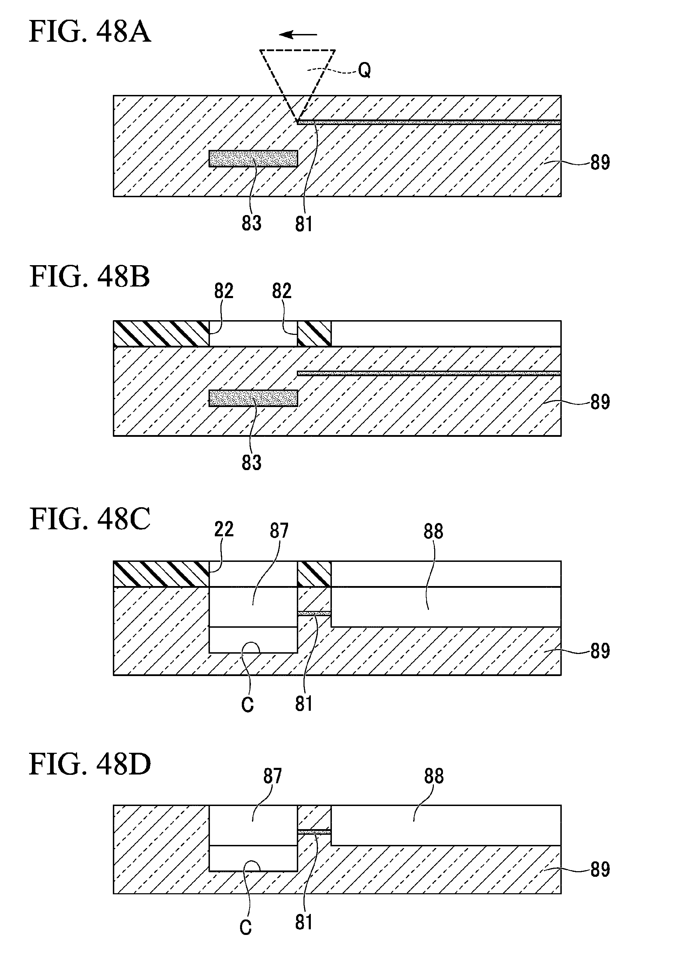

The above-mentioned base body 10A will be taken as an example to describe a fourth aspect of a manufacturing method according to the present invention. In this case, as shown in FIGS. 48A to 48D, the manufacturing method at least includes a process D1 of irradiating, at least, a region 81 in which a fine vacuum hole of a single member 89 is formed and a region 83 in which a slow flow portion P is formed, with a laser having a pulse duration of a picosecond order or less, to form a first modified region 81 and a third modified region 83 in each of the regions (FIG. 48A), a process D2 of forming a first channel 87 and the slow flow portion P in the single member 89 (FIG. 48B to D), and a process D3 of removing the modified region 81 from the single member 89 by etching.

A material of the member 89 may be the same as that of the member 59 described in the above-mentioned first aspect of the manufacturing method according to the present invention. In FIGS. 48A to 48D, the single member 89 is a transparent glass substrate (hereinafter, called the glass substrate 89).

### [Process D1]

The region 81 in which the fine vacuum hole of the single glass substrate 89 is formed and the region in which a third channel 88 is formed are irradiated with the laser Q having a pulse duration of a picosecond order or less, to form the first modified region 81 in these regions. Further, the third modified region 83 is also formed by irradiating the region 83 in which the recess portion C constituting the slow flow portion P is formed with a laser. Specifically, the above process may be performed similarly to the process A1 in the first aspect of the manufacturing method according to the present invention.

Similarly to the above-mentioned laser irradiation method α, it is required that in the laser irradiation conditions when the first modified region 81 is formed in the region in which the fine vacuum hole is formed, the laser polarization and the laser scanning direction are perpendicular to each other. On the other hand, in the laser irradiation conditions when the third modified region 83 is formed in the region to be the recess portion C, a method different from the laser irradiation method α may be used. In a case of conditions in which the region can be modified by laser irradiation, the conditions are not particularly limited, and a known method can be applied.

### [Process D2]

Next, a resist 82 is patterned and disposed on the upper surface of the glass substrate 89 by photolithography (FIG. 48B). Subsequently, a region in which the resist 82 on the upper surface of the glass substrate 89 is not disposed is eroded and removed by dry etching, wet etching, or a method such as sand blast until reaching a predetermined depth. At this time, the first modified region 81 formed in the region in which the third channel 88 is formed is removed by etching. In addition, the third modified region 83 formed in the region in which the recess portion C is formed is etched more rapidly than the rate at which a non-modified region to be the first channel 87 is etched. For this reason, the recess portion C can be formed simultaneously with the time when the first channel 87 is formed, or earlier than the time when the first channel 87 is formed (FIG. 48C).
In the manufacturing method of the fourth aspect, the region in which the recess portion C is formed is modified in advance, thereby allowing the recess portion C to be formed by etching at the time of etching for forming the first channel 87. That is, this is preferable because, after the first channel 87 is formed, the recess portion C is not required to be formed using etching of another process by disposing a mask of a resist again, thereby allowing the manufacturing process to be simplified.

When the resist 82 is peeled off, the first channel 87, the recess portion C, the third channel 88, and the glass substrate 89 in which the first modified region 81 is formed are obtained (FIG. 48D).

Further, in the process D2, in order to form the first channel 87 and the third channel 88, a drilling mechanism such as drill (laser drill) may be used or used in combination. When only the drilling mechanism is used, and the modified region 81 is etched in a process D3 described later, it is preferable to form a recess portion by etching the third modified region 83 similarly. In this case, this is also preferable because, after the first channel 87 is formed, the recess portion C is not required to be formed using etching of another process by disposing a mask of a resist again, thereby allowing the manufacturing process to be simplified.

### [Process D3]

Subsequently, the modified region 81 formed in the process D1 and the process D2 is removed from the single glass substrate 89 by etching (not shown).
As an etching method, wet etching is preferable. The modified region 81 having a cross-section exposed to the lateral side of the first channel 87 and the third channel 88 has a low etching resistance, and thus can be etched selectively or preferentially.
Specifically, the above process can be performed similarly to the process A2 in the first aspect of the manufacturing method according to the present invention.

As a result of the etching, the fine vacuum hole having a diameter of the opening on the order of nanometers can be formed at a predetermined position within the glass substrate 89 so as to communicate between the first channel 87 and the third channel 88.

Next, the upper surface of the first channel 87 and the upper surface of the third channel 88 can be formed by bonding the member to the upper surface of the glass substrate 89 so as to cover the first channel 87 and the third channel 88 which are formed (not shown).

A method of bonding the member to the upper surface of the glass substrate 89 may be performed using a known method depending on the material of the member 86.
In addition, at the time of the bonding, an electrode, a wiring or the like for an electrophysiological measurement can also be appropriately installed within the first channel 87 and the third channel 88.

A material of the member is not particularly limited, but a resin substrate such as PDMS and PMMA, or a glass substrate can be used. In addition, the member may be formed of a material that transmits light (for example, ray of visible light) used for observation, and may be formed of a material that does not transmit light. When only the trapping of the fine particle in intended, it is not necessarily required to transmit light used for observation. A member that transmits light used for observation may be preferable because the observation can be performed optically from the upper surface.

As stated above, each of the methods for manufacturing the base body having the recess portion C being disposed has been described. It is also possible to manufacture a base body having the recess portion D being disposed by the same method as the above. At this time, the recess portion D is formed as a portion of the channel. That is, when a resist is formed on the upper surface of the base member, the resist may preferably be formed in a portion of the lateral side of the channel by photolithography so the recess portion D having a desired shape is disposed.

### INDUSTRIAL APPLICABILITY

In a base body of the present invention and a method for manufacturing the base body, it is possible to trap a fine particle such as an microorganism or a cell contained in water or air, and to make wide use of the employment and manufacturing of a micro fluid device for performing various observations, analyses, and measurements.

### DESCRIPTION OF THE REFERENCE SYMBOLS

1: fine vacuum hole, 1a: first opening of fine vacuum hole, 1b: second opening of fine vacuum hole, C: recess portion, D: recess portion, e1: end of corner in recess portion, e2: first corner, e3: second corner, J: protruding portion, K1: electrode, K2: electrode, Q: laser light, M: objective lens of microscope, U1: protrusion, U2: protrusion, P: slow flow portion, S: adsorption portion, T: fine particle (microorganism or cell), R: fluid, L: depth, W: width, 3: channel, 3a: lateral side of channel, 3b: lower surface of channel, 3c: lateral side of channel, 3d: upper surface of channel, 4: base member, 6: substrate, 7: second channel, 8: third channel, 9, 9a, 9b, 9c: member, 10A, 10B, 10C, 10D, 10E, 10F, 10G, 10H: base body, 20A, 20B: base body, 11: fourth channel, 12: fifth channel, 30A, 30B: base body, 21: fine vacuum hole, 23: channel, 23a: lateral side of channel, 23b: lower surface of channel, 23c: lateral side of channel, 23d: upper surface of channel, 24: base member, 26: substrate, 28: third channel, 31: fourth channel, 32: fifth channel, 40A, 40B: base body, 51: region (first modified region) in which fine vacuum hole is formed, 52: region (second modified region) in which first channel is formed, 53: region (third modified region) in which slow flow portion is formed, 54: region in which second channel is formed, 55: fine vacuum hole, 56: member, 57: first channel, 58: third channel, 59: member (glass substrate), 61: modified region, 62: resist, 65: fine vacuum hole, 67: first channel, 68: third channel, 69: member (glass substrate), 101: cell, 104: electrode, 105: electrode, 108: resin well, 110: resin substrate, 112: resin substrate

## Claims

1. A base body comprising:
a base member;
a channel provided in the base member, the cannel having an inner wall surface and circulating a fluid;
a fine vacuum hole provided in the inner wall, the fine vacuum hole causing the channel to communicate with the outside of the base member and having an opening; and
a slow flow portion disposed at a position close to the opening of the fine vacuum hole in the inner wall surface, the slow flow portion slowing a flow of the fluid, wherein
at least a portion that configures the fine vacuum hole in the base member is formed of a single member.

2. The base body according to Claim 1, wherein
the inner wall surface of the channel comprises a first inner wall surface in which the slow flow portion is disposed and a second inner wall surface in which the opening of the fine vacuum hole is disposed, and
the first inner wall surface and the second inner wall surface are different from each other.

3. The base body according to Claim 1, wherein
the inner wall surface of the channel comprises a first inner wall surface in which the slow flow portion is disposed and a second inner wall surface in which the opening of the fine vacuum hole is disposed, and
the first inner wall surface is the same as the second inner wall surface.

4. The base body according to any one of claims 1 to 3, wherein the slow flow portion is a recess portion formed in the inner wall surface.

5. The base body according to Claim 4, further comprising:
a protruding portion provided at a position close to the recess portion, the protruding portion protruding from an inner wall surface different from the inner wall surface in which the fine vacuum hole is formed.

6. The base body according to Claim 4 or 5, wherein
the recess portion comprises a first corner provided at a position close to the opening and a second corner provided at a position away from the opening, and
in a longitudinal cross-section of the fine vacuum hole along an extending direction of the channel, when a distance between the first corner in a direction perpendicular to the extending direction of the channel and the opening is denoted by L, and a distance between the first corner in the extending direction of the channel and the opening is denoted by W, a ratio of L/W is in a range of 0.05 to 100.

7. A method for manufacturing a base body, comprising:
preparing a base member comprising a single member;
forming a first modified region by irradiating a region in which a fine vacuum hole is formed, in the single member, with a laser having a pulse duration of a picosecond order or less;
forming a second modified region by irradiating a region in which a channel is formed, in the single member, with a laser having a pulse duration of a picosecond order or less;
forming a third modified region by irradiating a region in which a slow flow portion is formed, in the single member, with a laser having a pulse duration of a picosecond order or less; and
removing the first modified region, the second modified region, and the third modified region from the single member by etching.

8. A method for manufacturing a base body, comprising:
preparing a base member comprising a single member;
forming a channel and a slow flow portion in the single member;
forming a modified region by irradiating a region in which a fine vacuum hole is formed, in the single member, with a laser having a pulse duration of a picosecond order or less; and
removing the modified region from the single member by etching.

9. A method for manufacturing a base body, comprising:
preparing a base member comprising a single member;
forming a modified region by irradiating, at least, a region in which a fine vacuum hole is formed and a portion of a region in which a channel is formed, in the single member, with a laser having a pulse duration of equal to or less than one picosecond;
forming the channel and the slow flow portion in the single member; and
removing the modified region from the single member by etching.

10. A method for manufacturing a base body, comprising:
preparing a base member comprising a single member;
forming a modified region by irradiating, at least, a region in which a fine vacuum hole is formed and a region in which a slow flow portion is formed, in the single member, with a laser having a pulse duration of a picosecond order or less;
forming the channel and the slow flow portion in the single member; and
removing the modified region from the single member by etching.
